# EUROPEAN PATENT APPLICATION

(11) **EP 4 450 842 A1**
(43) Date of publication of application: **23.10.2024**
(21) Application number: 22907522.1
(22) Date of filing: 15.12.2022
(51) Int. Cl.: F16D 41/08, F16D 11/08, F16H 3/08

(54) **INTERRUPTING DEVICE AND COUPLING DEVICE**

(30) Priority: 15.12.2021 JP 2021203497
(71) Applicant: HONDA MOTOR CO., LTD., Minato-ku Tokyo 107-8556 (JP)
(72) Inventor: SHIMADA, Kei, Wako-shi, Saitama 351-0193 (JP); KOTANI, Yoshiaki, Wako-shi, Saitama 351-0193 (JP); ONOZAWA, Seiji, Wako-shi, Saitama 351-0193 (JP); ONO, Hiromi, Wako-shi, Saitama 351-0193 (JP); HIKI, Yutaka, Wako-shi, Saitama 351-0193 (JP)
(74) Representative: Weickmann & Weickmann PartmbB
(86) International application number: PCT/JP2022/046288
(87) International publication number: WO 2023/112999

(57) **Abstract**

A first interrupting mechanism (210) comprises a roller (281) disposed between a first driven gear (184) and a second shaft (182) and an operating rod (241) that operates the roller (281) between an engagement state in which the first driven gear (184) and the second shaft (182) can be rotated integrally with each other and a non-engagement state in which the first driven gear (184) and the second shaft (182) can be rotated relative to each other. The operating rod (241) can operate the roller (281) via a retainer (282) and abuts against the inner end of a pin (283).

## Description

### TECHNICAL FIELD

The present invention relates to an connection and disconnection device and a joint device.

### BACKGROUND ART

In the related art, there is known a connection and disconnection device that switches between a state in which rotors are integrally rotatable and a state in which the rotors are relatively rotatable. Such a connection and disconnection device is used for a drive device of a vehicle, a joint device, and the like (for example, Patent Literature 1).

### CITATION LIST

### PATENT LITERATURE

Patent Literature 1: WO2021/040039

### SUMMARY OF INVENTION

### TECHNICAL PROBLEM

A connection and disconnection device is required to be capable of appropriately switching between the two states depending on a situation.

The present invention provides a connection and disconnection device and a joint device capable of appropriately switching between a state in which rotors are integrally rotatable and a state in which rotors are relatively rotatable.

### SOLUTION TO PROBLEM

According to an aspect of the present invention, there is provided a connection and disconnection device, including:
an engagement element disposed between a first rotation member and a second rotation member; and
an operation unit configured to operate the engagement element to be in an engagement state in which the first rotation member and the second rotation member are integrally rotatable or a non-engagement state in which the first rotation member and the second rotation member are relatively rotatable, in which
the operation unit includes
   an actuator configured to move the engagement element, and
   an operator configured to operate the engagement element via the actuator or to operate the engagement element not via the actuator,
the first rotation member and the second rotation member are arranged such that
   rotation axes of the first rotation member and the second rotation member coincide with each other, and
   the first rotation member and the second rotation member at least partially overlap each other when viewed in an orthogonal direction orthogonal to the rotation axes,
the operator includes
   an advancing and retracting element that is provided to be capable of advancing and retracting along the orthogonal direction orthogonal to the rotation axes, and
   an extension portion that extends along the rotation axes and is provided to be capable of advancing and retracting along the rotation axes, and
the advancing and retracting element is provided such that an inner end thereof, which is an end on a side closer to the rotation axes in the orthogonal direction, is in contact with the extension portion.

According to another aspect of the present invention, there is provided a joint device with the above connection and disconnection device, the joint device including:
a first member;
a second member;
a connecting unit that connects the first member and the second member such that an angle formed between the first member and the second member is changeable; and
an enlarging and reducing device configured to enlarge and reduce the angle formed between the first member and the second member, in which
the enlarging and reducing device includes a power source and a power transmission unit configured to transmit power of the power source, and
the connection and disconnection device is applied to the power transmission unit.

### ADVANTAGEOUS EFFECTS OF INVENTION

According to the present invention, it is possible to appropriately switch between a state in which rotors are integrally rotatable and a state in which the rotors are relatively rotatable.

### BRIEF DESCRIPTION OF DRAWINGS

FIG. 1 is a perspective view of an electric prosthetic leg 1 equipped with a connection and disconnection device according to an embodiment of the present invention, as viewed obliquely from a front side.
FIG. 2 is an exploded perspective view of the electric prosthetic leg 1 in FIG. 1.
FIG. 3 is a perspective view of an expansion device 140.
FIG. 4 is a cross-sectional view of the electric prosthetic leg 1 in FIG. 1.
FIG. 5 is a cross-sectional view of the expansion device.
FIG. 6 is a cross-sectional view of a main part showing a bending state of the electric prosthetic leg 1 in FIG. 1.
FIG. 7 is a cross-sectional view of a main part showing a maximum bending state of the electric prosthetic leg 1 in FIG. 1.
FIG. 8 is a cross-sectional view of a two-way clutch 280.
FIG. 9 is a perspective view showing an example (including rollers 281, guides 284, and rubber balls 282c) of a retainer 282 shown in FIG. 8.
FIG. 10 is a perspective view showing another example (including rollers 281 and rubber balls 282c) of the retainer shown in FIG. 8.
FIG. 11 is a diagram showing an operation of an operation mechanism 240, in which (A) shows a state in which a first interrupting unit 212 and a second interrupting unit 222 are in an off state, and (B) shows a state in which the first interrupting unit 212 is in the off state and the second interrupting unit 222 is in an on state, and (C) shows a state in which the first interrupting unit 212 is in the on state and the second interrupting unit 222 is in the off state.
In FIG. 12, (A) is a cross-sectional view showing a state in which the second interrupting unit 222 is in the off state, and (B) is diagram showing a position of an operation rod 241 in the above case.
In FIG. 13, (A) is a cross-sectional view showing a state in which the second interrupting unit 222 is operated from the off state to the on state, and (B) is diagram showing a position of the operation rod 241 in the above case.
In FIG. 14, (A) is a cross-sectional view showing a normal rotation on state of the second interrupting unit 222, and (B) is diagram showing a position of the operation rod 241 in the above case.
In FIG. 15, (A) is a cross-sectional view showing a reverse rotation on state of the second interrupting unit 222, and (B) is diagram showing a position of the operation rod 241 in the above case.
In FIG. 16, (A) is a cross-sectional view showing a state in which the second interrupting unit 222 is operated from the on state to the off state, and (B) is diagram showing a position of the operation rod 241 in the above case.
FIG. 17 is a diagram showing operations (stair ascending operations) of a user and the electric prosthetic leg 1 when ascending stairs.
FIG. 18 is a diagram showing operations (level ground walking operations) of the user and the electric prosthetic leg 1 when walking on a level ground.
FIG. 19 is a cross-sectional view showing a coupling portion between the operation rod 241 and a rack 241a in the electric prosthetic leg 1 of a first modification.
FIG. 20 is a perspective view of a drive unit of the electric prosthetic leg 1 of a second modification.
FIG. 21 is an exploded perspective view of the drive unit of the electric prosthetic leg 1 of the second modification.
FIG. 22 is a schematic diagram illustrating an operation of the drive unit of the electric prosthetic leg 1 of the second modification in a state where the rollers 281 are in a non-engagement state.
FIG. 23 is a schematic diagram illustrating an operation of the drive unit of the electric prosthetic leg 1 of the second modification when the rollers 281 transition from the engagement state to the non-engagement state.
FIG. 24 is a graph showing a current value of a motor (motor current), a position of a rod, and an elastic energy in the non-engagement state of the rollers 281, during transition from an engagement state to the non-engagement state of the rollers 281, and during transition from the engagement state to the non-engagement state of the rollers 281 (without an elastic energy accumulating mechanism 300).
FIG. 25 is a perspective view of an elastic energy accumulating mechanism 300A.
FIG. 26 is a schematic diagram illustrating an operation of the elastic energy accumulating mechanism 300A when the rollers 281 are in the non-engagement state.
FIG. 27 is a schematic diagram illustrating an operation of the elastic energy accumulating mechanism 300A when the rollers 281 are in the engagement state.
FIG. 28 is a perspective view of the drive unit in a third modification.
FIG. 29 is an exploded perspective view of the drive unit in the third modification.
FIG. 30 is a diagram illustrating an operation of the drive unit in the third modification.
FIG. 31 is a cross-sectional view of the drive unit in a fourth modification.
FIG. 32 is another cross-sectional view of the drive unit in the fourth modification.
FIG. 33 is a diagram illustrating a Geneva mechanism.
FIG. 34 is a diagram illustrating an operation of the drive unit in a fourth modification.
FIG. 35 is a perspective view of the drive unit in a fifth modification.
FIG. 36 is a diagram in which a cam groove 711 formed in a cam drum 710 is developed in a rotation direction.
FIG. 37 is a schematic diagram of a vehicle drive device 900 equipped with the connection and disconnection device according to an embodiment of the present invention.

### DESCRIPTION OF EMBODIMENTS

Hereinafter, an electric prosthetic leg quipped with a connection and disconnection device according to an embodiment of the present invention will be described with reference to the drawings. Note that in the following description, a front-rear direction, a left-right direction, and an upper-lower direction are defined with reference to a user of the electric prosthetic leg. In the drawings, a front side of the electric prosthetic leg is denoted by Fr, a rear side is denoted by Rr, a left side is denoted by L, a right side is denoted by R, an upper side is denoted by U, and a lower side is denoted by D.

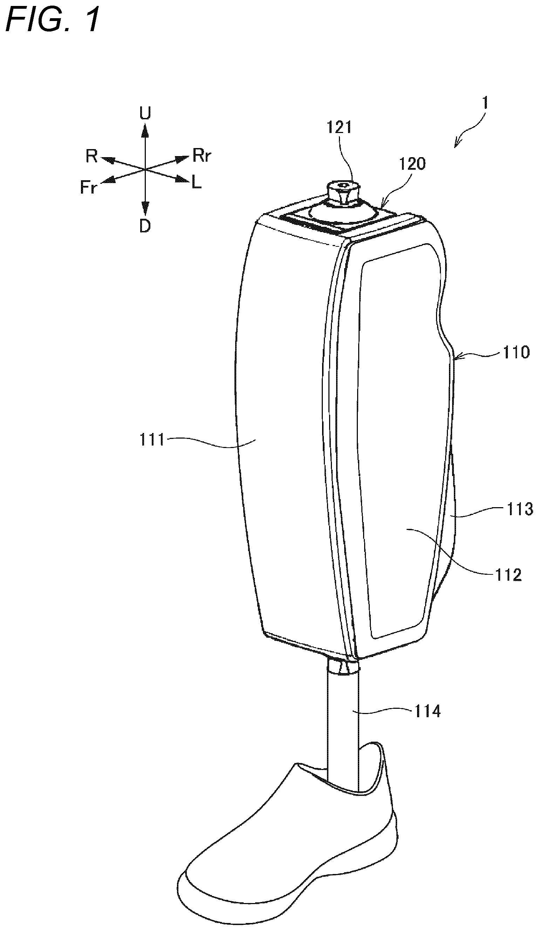

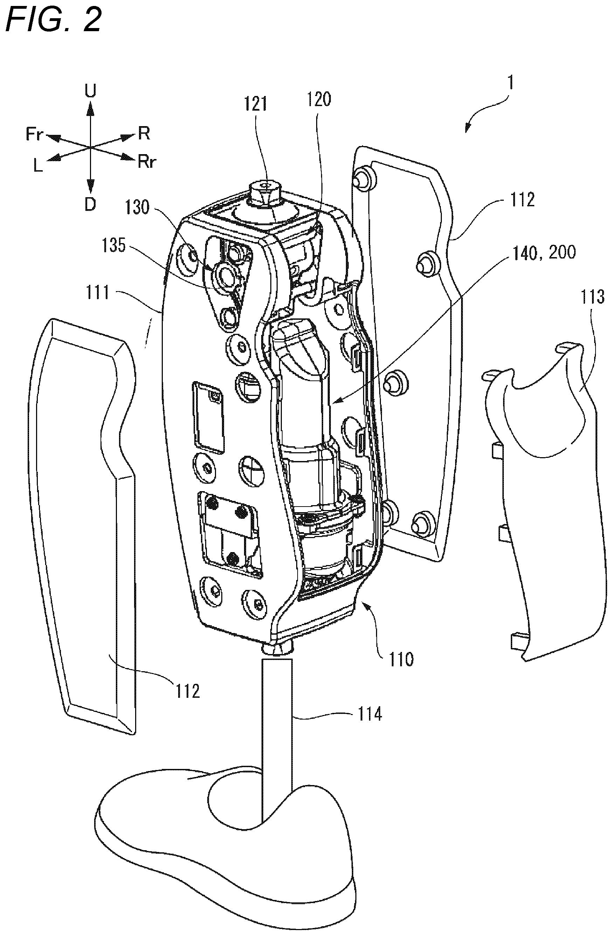

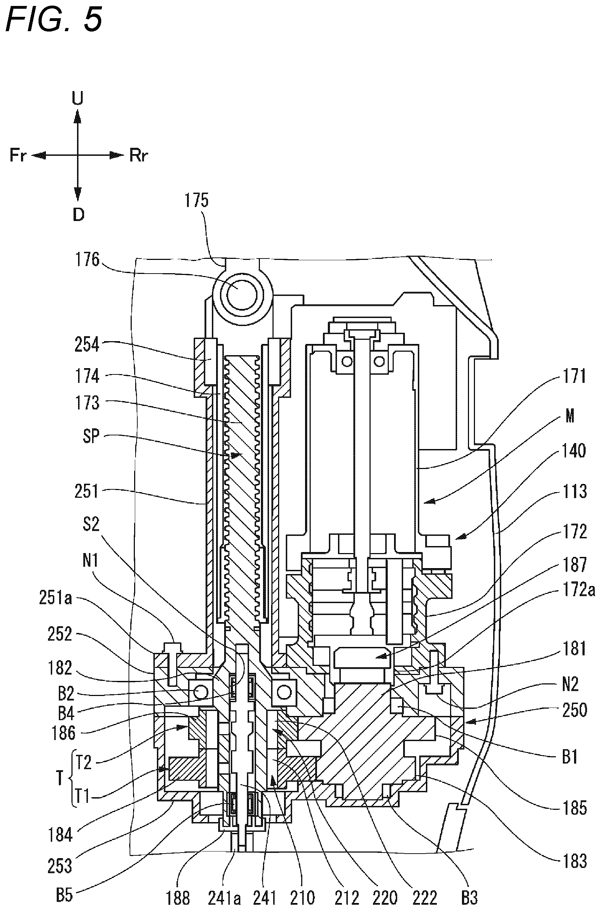

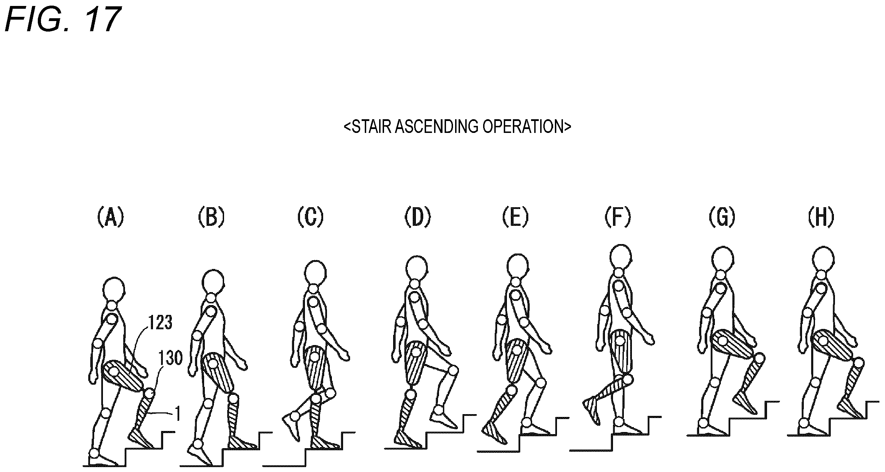

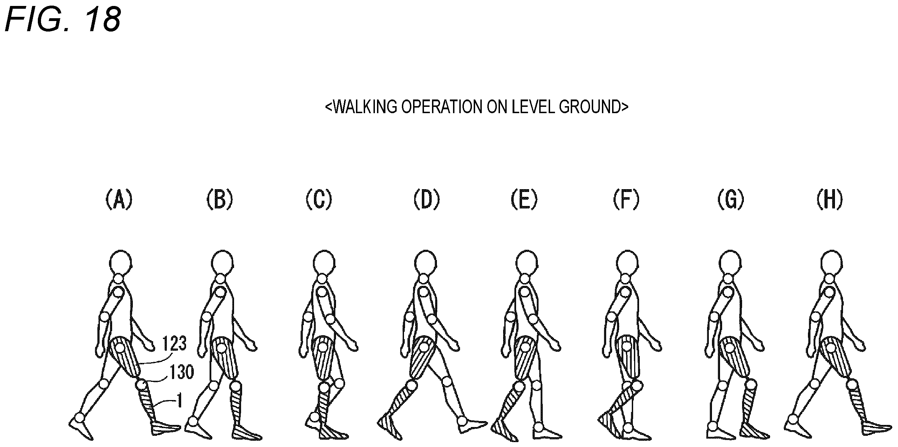

As shown in FIGS. 1 to 5, an electric prosthetic leg 1 according to the present embodiment is a prosthetic leg that is attached to a leg of a person who does not have a knee. The electric prosthetic leg 1 includes: a below-knee member 110 positioned on a lower side of the knee, an above-knee member 120 positioned on an upper side of the knee and attached to a thigh 123 (refer to FIGS. 17 and 18), a knee joint mechanism 130 that connects the below-knee member 110 and the above-knee member 120 such that an angle formed therebetween is changeable, an enlarging and reducing device 200 capable of enlarging and reducing the angle formed between the below-knee member 110 and the above-knee member 120, a mechanical stop mechanism 150 that mechanically limits a range of change in the angle formed between the below-knee member 110 and the above-knee member 120, a buffer mechanism 160 that buffers an impact caused by the mechanical stop mechanism 150, and a battery B that supplies power to the enlarging and reducing device 200 and the like.

The above-knee member 120 includes an adapter 121 coupled to a socket (not shown). The socket is a joint member provided on the thigh 123, and the above-knee member 120 is integrated with the thigh 123 by coupling the adapter 121 to the socket.

The below-knee member 110 includes a box-shaped main frame 111 with open upper portion and rear portion, a side cover 112 that covers both left and right side surfaces of the main frame 111, and a detachable rear cover 113 that covers a rear opening of the main frame 111 in an openable and closable manner.

The above-knee member 120 is provided on the upper portion of the main frame 111 through a pivoting portion 135 that constitutes the knee joint mechanism 130, and a leg 114 extending to the lower side is provided on a lower portion of the main frame 111.

An enlarging and reducing device 200 capable of enlarging and reducing the angle formed between the above-knee member 110 and the below-knee member 120 is provided in a space formed by the below-knee member 120 and the above-knee member 110. The enlarging and reducing device 200 of the present embodiment is an expansion device 140 capable of enlarging and reducing the angle formed between the below-knee member 110 and the above-knee member 120 by expanding and contracting. The expansion device 140 extends in the upper-lower direction, which will be described later in detail, and is mechanically connected to the above-knee member 120 on one side in the extending direction and mechanically connected to the below-knee member 110 on the other side in the extending direction. Note that the term "mechanically connected" is a concept that includes a configuration of direct connection and a configuration of connection through another member.

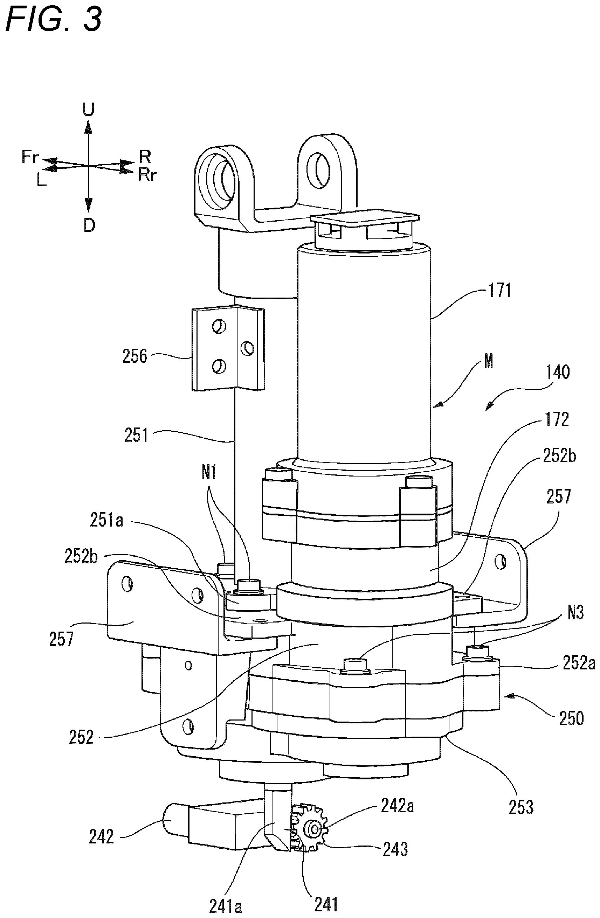

As shown in FIGS. 3 to 5, the expansion device 140 includes: a motor M that outputs rotation power, a transmission T that transmits the power of the motor M, a spindle unit SP that is connected to the transmission T in a manner of being capable of transmitting power and converts the rotation power output from the transmission T into translation motion (expanding and contracting motion), a first interrupting mechanism 210 and a second interrupting mechanism 220 provided in the transmission T, and a unit case 250 that unitizes the expansion device 140.

The motor M is disposed on the rear side and upper side of the transmission T, and the spindle unit SP is disposed on the front side and upper side of the transmission T. The motor M is a motor with a built-in gear mechanism, including a motor body 171 and a gear mechanism unit 172 that decelerates output rotation of the motor body 171. The spindle unit SP includes a spindle 173 formed with a male screw and a sleeve 174 formed with a female screw, and rotation of the spindle 173 causes the sleeve 174 to translate along an axis of the spindle 173.

Specifically, the spindle 173 rotates after receiving the rotation power of the motor M transmitted by the transmission T. On the other hand, the sleeve 174 is non-rotatably and movably in the upper-lower direction supported by the unit case 250. Therefore, when the spindle 173 rotates to one side after receiving the rotation power of the motor M transmitted by the transmission T, the sleeve 174 is translated away from the transmission T, and when the spindle 173 rotates to the other side, the sleeve 174 is translated in a direction of approaching the transmission T. Note that the translation motion of the sleeve 174 away from the transmission T may be referred to as an expanding operation of the spindle unit SP, and conversely, the translation motion of the sleeve 174 approaching the transmission T may be referred to as a contracting operation of the spindle unit SP.

That is, a distance between the sleeve 174 and the transmission T increases or decreases depending on a rotation direction of the spindle 173. An upper end of the sleeve 174 is coupled to the above-knee member 120 through a link member 175. As the distance between the sleeve 174 and the transmission T increases or decreases in accordance with the rotation direction of the spindle 173, the below-knee member 110 and the above-knee member 120 rotate about the pivoting portion 135. Accordingly, the angle formed between the above-knee member 120 and the below-knee member 110 changes. If the angle formed between the above-knee member 120 and the below-knee member 110 is an acute angle between an acute angle and an obtuse angle, the knee joint mechanism 130 stretches when the angle becomes large, and the knee joint mechanism 130 bends when the angle becomes small.

Note that the enlarging and reducing device 200 of the present embodiment makes the expansion device 140 expand and contract by converting the rotation motion into the translation motion by the spindle unit SP of the expansion device 140, and thereby enlarging and reducing the angle between the below-knee member 110 and the above-knee member 120. However, instead of a portion that expands and contracts like the expansion device 140 (spindle unit SP), a gear meshing mechanism (meshing mechanism between spur gears, hypoid gear mechanism (registered trademark), worm gear mechanism, and the like) may be provided between the below-knee member 110 and the above-knee member 120 to enlarge and reduce the angle formed between the below-knee member 110 and the above-knee member 120 by transmitting the rotation motion.

The transmission T includes a first transmission mechanism T1 that transmits the power of the motor M to the spindle unit SP at a first transmission ratio, and a second transmission mechanism T2 that transmits the power of the motor M to the spindle unit SP at a second transmission ratio, which is different from the first transmission ratio. The first transmission mechanism T1 and the second transmission mechanism T2 are switched between a cutoff state and a power connection state by the interrupting mechanisms 210 and 220.

According to such a transmission T, by providing two power transmission paths with different transmission ratios, it is possible to switch operating speed and generated power for stretching and bending in the knee joint mechanism 130. One of the first transmission mechanism T1 and the second transmission mechanism T2 may be a speed reduction mechanism and the other may be a speed increasing mechanism, or one may be a constant speed mechanism and the other may be a speed reduction mechanism or a speed increasing mechanism, or both may be speed reduction mechanisms, or both may be speed increasing mechanisms, as long as the first transmission ratio is different from the second transmission ratio.

The first transmission ratio is a post-transmission rotation speed, which is a rotation speed on a side opposite to the motor M (spindle unit SP side) in the first transmission mechanism T1, with respect to a pre-transmission rotation speed, which is a rotation speed on the motor M side in the first transmission mechanism T1. The second transmission ratio is a post-transmission rotation speed, which is a rotation speed on the side opposite to the motor M (spindle unit SP side) in the second transmission mechanism T2, with respect to a pre-transmission rotation speed, which is a rotation speed on the motor M side in the second transmission mechanism T2.

For example, when the first transmission ratio of the first transmission mechanism T1 is smaller than 1, the rotation speed on the side opposite to the motor M (spindle unit SP side) becomes lower than the rotation speed on the motor M side, and a torque increases. When the second transmission ratio of the second transmission mechanism T2 is greater than 1, the rotation speed on the side opposite to the motor M (spindle unit SP side) becomes higher than the rotation speed on the motor M side, and a torque decreases. In the present embodiment, the first transmission ratio is set to be smaller than 1 and the second transmission ratio is set to be greater than 1, and the first transmission mechanism T1 is disposed below the second transmission mechanism T2.

The first transmission mechanism T1 and the second transmission mechanism T2 include a first shaft 181 rotatably disposed on a downward extension line of an output shaft 172a of the gear mechanism unit 172, and a second shaft 182 rotatably disposed on a downward extension line of the spindle 173 of the spindle unit SP. The first shaft 181 is coupled to the output shaft 172a of the gear mechanism unit 172 of the motor M in a manner of being integrally rotatable through a coupling 187 that allows a shaft center error. The second shaft 182 is connected to the spindle 173 of the spindle unit SP in a manner of being integrally rotatable. Note that although the second shaft 182 of the present embodiment is integrated with the spindle 173 of the spindle unit SP, the second shaft 182 may be coupled to the spindle 173 of the spindle unit SP by spline fitting or coupling.

The first transmission mechanism T1 includes a first drive gear 183 and a first driven gear 184 that mesh with each other. The first drive gear 183 is integrally rotatably supported by the first shaft 181, and the first driven gear 184 is relatively rotatably supported by the second shaft 182 positioned in an internal space of the first driven gear 184. The first driven gear 184 and the second shaft 182 have the same rotation axis. When viewed in an orthogonal direction orthogonal to the rotation axis, the first driven gear 184 and the second shaft 182 are disposed so as to overlap each other at least partially. In other words, at least a part of each is disposed on the same plane orthogonal to the rotation axis. The first transmission mechanism T1 of the present embodiment is a deceleration transmission mechanism in which the first drive gear 183 has a diameter smaller than that of the first driven gear 184, and can make the spindle unit SP expand and contract at low speed and high torque.

The second transmission mechanism T2 includes a second drive gear 185 and a second driven gear 186 that mesh with each other. The second drive gear 185 is integrally rotatably supported by the first shaft 181, and the second driven gear 186 is relatively rotatably supported by the second shaft 182 positioned in an internal space of the second driven gear 186. The second driven gear 186 and the second shaft 182 have the same rotation axis. When viewed in an orthogonal direction orthogonal to the rotation axis, the second driven gear 186 and the second shaft 182 are disposed so as to overlap each other at least partially. In other words, at least a part of each is disposed on the same plane orthogonal to the rotation axis. The second transmission mechanism T2 of the present embodiment is an acceleration transmission mechanism in which the second drive gear 185 has a diameter greater than that of the second driven gear 186, and can make the spindle unit SP expand and contract at high speed and low torque. Note that in the present embodiment, the second transmission mechanism T2 is disposed above the first transmission mechanism T1, but the second transmission mechanism T2 may be disposed below the first transmission mechanism T1. That is, the first driven gear 184 and the second driven gear 186 may be at different positions in the rotation axis direction. Although the first shaft 181 and the second shaft 182 of the present embodiment are integrally formed from the beginning, the first shaft 181 and the second shaft 182 may be integrally coupled (combined) after upper and lower gear support portions are formed as separate bodies.

The first interrupting mechanism 210 includes the first interrupting unit 212 provided between the first driven gear 184 and the second shaft 182. The second interrupting mechanism 220 includes the second interrupting unit 222 provided between the second driven gear 186 and the second shaft 182. These interrupting units 212 and 222 have a common configuration, and are configured to be switchable between the cutoff state in which power transmission is cut off, and a power transmittable state in which rotation power in both one direction and the other direction can be transmitted. Details of the interrupting units 212 and 222 will be described later.

As shown in FIGS. 3 to 5, the unit case 250 includes an upper case 251, a middle case 252, and a lower case 253.

The upper case 251 has a cylindrical shape covering an outer peripheral side of the spindle unit SP, and supports the sleeve 174 of the spindle unit SP through a bush 254 provided on an inner peripheral side of an upper end thereof in a manner of being non-rotatable and movable in the upper-lower direction. A flange 251a extending outward is provided at a lower end of the upper case 251. The upper case 251 is fastened to a front side and an upper side of the middle case 252 by a plurality of screws N1 penetrating the flange 251a from above.

The middle case 252 rotatably supports an upper end side of the first shaft 181 through a bearing B1, and rotatably supports an upper end side of the second shaft 182 through a bearing B2. The upper case 251 is fastened to the front side and the upper side of the middle case 252, and the motor M is fastened to a rear side and an upper side of the middle case 252. The motor M is fastened to the middle case 252 by a plurality of screws N2 penetrating the middle case 252 from the lower side (inner side). A lower flange 252a for fastening the lower case 253 and a pair of upper flanges 252b for fixing to the main frame 111 are provided on the outer peripheral portion of the middle case 252.

The lower case 253 is fastened to a lower side of the middle case 252 by a plurality of screws N3 penetrating the lower flange 252a of the middle case 252 from above. The lower case 253 not only covers a lower side and lateral sides of the transmission T, but also rotatably supports a lower end side of the first shaft 181 through a bearing B3.

According to such a unit case 250, with the three-stage structure of the upper case 251, the middle case 252, and the lower case 253, not only the transmission T and the spindle unit SP can be housed, but also the expansion device 140 including the motor M can be unitized, so that the number of parts and weight can be reduced.

As shown in FIG. 3, the unit case 250 is attached to the main frame 111 through one upper bracket 256 and a pair of middle brackets 257. The upper bracket 256 supports the upper end of the upper case 251 on a front wall of the main frame 111, and the pair of middle brackets 257 support the pair of upper flanges 252b formed on both left and right side portions of the middle case 252 on left and right side walls of the main frame 111.

For example, in a case where the upper bracket 256 and the middle brackets 257 are attached to the main frame 111 side and then the expansion device 140 is assembled, the expansion device 140 can be temporarily held by the main frame 111 by placing the pair of upper flanges 252b of the middle case 252 on the pair of middle brackets 257, and thus the work of fastening the middle case 252 to the middle brackets 257 and the work of fastening the upper case 251 to the upper bracket 256 are facilitated. An operation of detaching the expansion device 140 according to a reverse procedure also becomes easy.

Since the upper case 251 and the middle cases 252 receive a higher load than the lower case 253, by fastening the upper case 251 and the middle cases 252 to the main frame 111 through the upper bracket 256 and the middle brackets 257, not only support strength of the transmission T and the spindle unit SP is increased, but also rigidity of the lower case 253 is reduced, thereby achieving weight reduction.

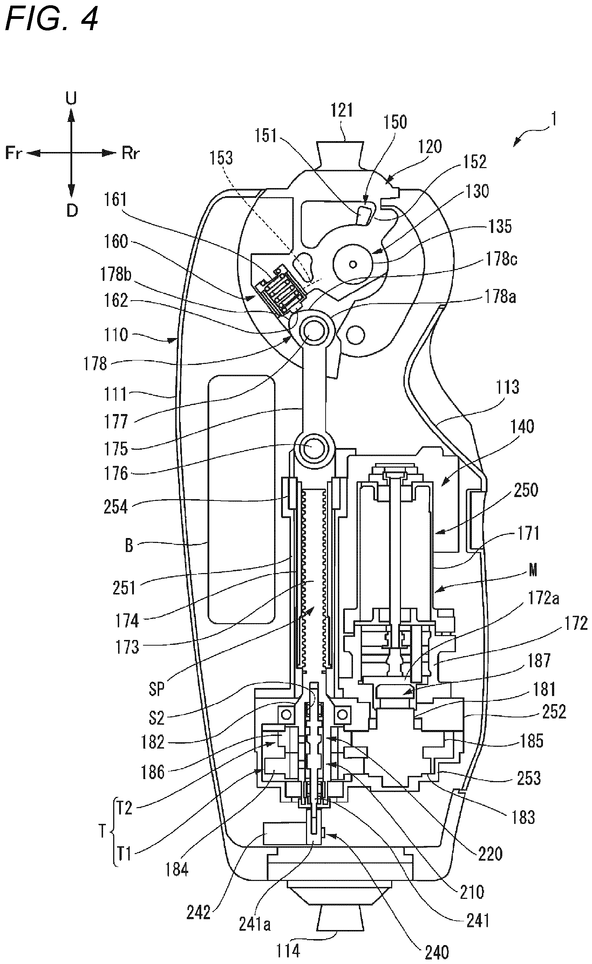

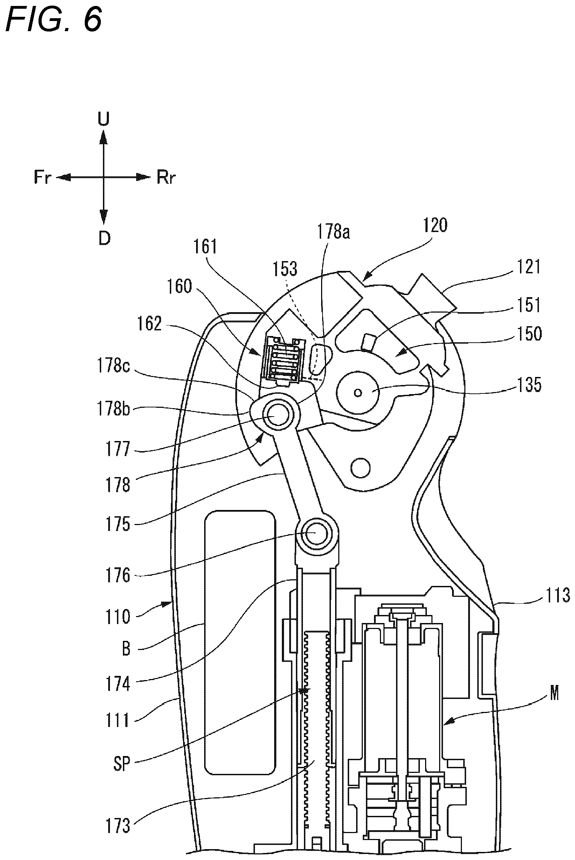

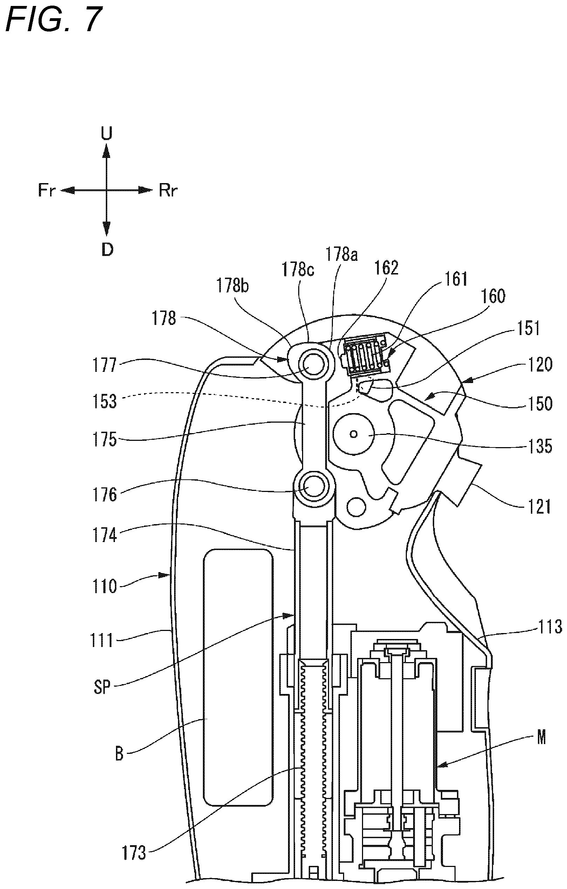

FIG. 4 shows a stretching state of the electric prosthetic leg 1, and FIG. 6 shows a bending state of the electric prosthetic leg 1, and FIG. 7 shows a maximum bending state of the electric prosthetic leg 1. Note that in walking by the electric prosthetic leg 1, the maximum bending state shown in FIG. 7 does not occur.

As shown in FIGS. 4, 6, and 7, the mechanical stop mechanism 150 includes a stopper member 151 provided on the below-knee member 110, and a first contact portion 152 and a second contact portion 153 provided on the above-knee member 120. In the state shown in FIG. 4, the first contact portion 152 comes into contact with the stopper member 151, thereby preventing the knee joint mechanism 130 from bending to the opposite direction. In the state shown in FIG. 7, the second contact portion 153 comes into contact with the stopper member 151, thereby preventing the knee joint mechanism 130 from further bending from the maximum bending state.

The buffer mechanism160 is provided on the above-knee member 120 side, and includes a pressing portion 162 capable of pressing an upper end of the link member 175 by a biasing force of a spring 161 (for example, a compression coil spring). A lower end of the link member 175 is rotatably coupled to the sleeve 174 of the spindle unit SP through a first pivoting portion 176, and the upper end of the link member 175 is rotatably coupled to the above-knee member 120 through a second pivoting portion 177. A cam portion 178 is formed at the upper end of the link member 175. The cam portion 178 includes a small diameter outer peripheral portion 178a having a small diameter and centered on the second pivoting portion 177, a large diameter outer peripheral portion 178b with a long distance from the second pivoting portion 177, and a coupling outer peripheral portion 178c that couples the small diameter outer peripheral portion 178a and the large diameter outer peripheral portion 178b without any step.

As shown in FIGS. 6 and 7, in a state where the knee joint mechanism 130 is bent, the pressing portion 162 faces the small diameter outer peripheral portion 178a of the cam portion 178, and thus the pressing portion 162 and the cam portion 178 are separated from each other. As shown in FIG. 4, when the knee joint mechanism 130 is stretched in accordance with a contracting operation of the spindle unit SP and approaches a mechanical stop position on the stretching side, a position to which the pressing portion 162 and the cam portion 178 face moves from the coupling outer peripheral portion 178c to the large diameter outer peripheral portion 178b, and the large diameter outer peripheral portion 178b of the cam portion 178 comes into contact with the pressing portion 162 and pushes the pressing portion 162 against the biasing force of the spring 161. In other words, the cam portion 178 is pressed in a return direction by the biasing force of the spring 161. As a result, the biasing force of the spring 161 acts as resistance, and the impact when the first contact portion 152 comes into contact with the stopper member 151 is buffered.

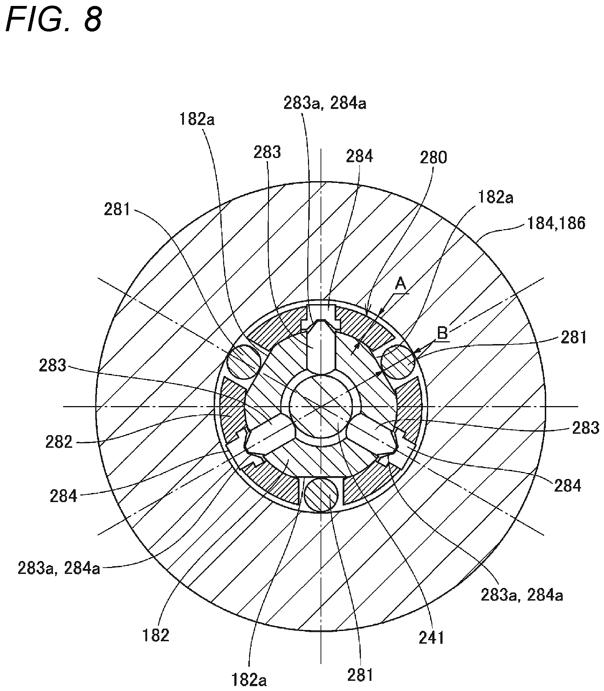

Next, details of the interrupting units 212 and 222 and the operation mechanism 240 will be described with reference to FIG. 8 and subsequent drawings.

Each of the interrupting units 212 and 222 has a common configuration, and is configured to be switchable between the cutoff state in which power transmission is cut off, and the power transmittable state in which rotation power in both one direction and the other direction can be transmitted. The interrupting units 212 and 222 of the present embodiment are configured using a two-way clutch 280 with a forced free function, as shown in FIG. 8. The two-way clutch 280 includes a plurality of (three in the present embodiment) rollers 281 arranged between an outer peripheral surface of the second shaft 182 and inner peripheral surfaces of the gears 184 and 186, a retainer 282 that holds the plurality of rollers 281 at predetermined intervals, an operation mechanism 240, a plurality of (three in the present embodiment) pins 283 that penetrate the second shaft 182 in a radial direction and are operated by the operation mechanism 240 to a forced free position and a forced free release position, and a plurality of (three in the present embodiment) guides 284 provided on the retainer 282 and defining a relative rotation position of the retainer 282 with respect to the second shaft 182 when the pins 283 are in the forced free position. The rollers 281 may be balls or sprags.

A distance A in the radial direction between the outer peripheral surface of the second shaft 182 and the inner peripheral surfaces of the gears 184 and 186 is smaller than a diameter B of the rollers 281. Flat portions 182a are formed on the outer peripheral portion of the second shaft 182 at predetermined intervals in a circumferential direction, and on a center side in the circumferential direction of the flat portions 182a, the distance A is larger than the diameter B.

In other words, when the rollers 281 are held at center portions of the flat portions 182a in the circumferential direction, the rollers 281 do not mesh with the outer peripheral surface of the second shaft 182 and the inner peripheral surfaces of the gears 184 and 186 (non-engagement state), and relative rotation between the second shaft 182 and the gears 184 and 186 is allowed (forced free state).

On the other hand, when the rollers 281 are allowed to move in the circumferential direction relative to the second shaft 182, the rollers 281 mesh with the outer peripheral surface of the second shaft 182 and the inner peripheral surfaces of the gears 184 and 186 (engagement state), and the second shaft 182 and the gears 184 and 186 are connected in a manner of being rotatable integrally in two directions (forced free release state).

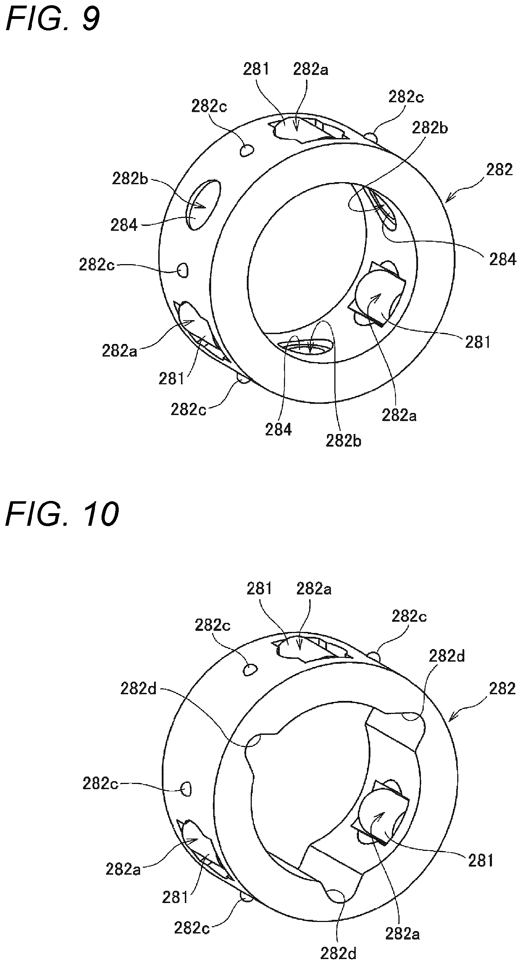

As shown in FIG. 9, the retainer 282 includes a plurality of roller holding portions 282a that are ring-shaped and capable of relative rotation with respect to the second shaft 182 and the gears 184 and 186, and that hold the rollers 281, and a plurality of guide holding portions 282b that hold the guides 284. In other words, the retainer 282 is provided adjacent to the rollers 281 in the circumferential direction with respect to the rotation axis.

A plurality of rubber balls 282c are embedded in an outer peripheral surface of the retainer 282 at predetermined intervals in the circumferential direction. These rubber balls 282c prevent unintended idling in the forced free release state by generating moderate friction between the gears 184 and 186 and the retainer 282. Note that the members for generating friction between the gears 184 and 186 and the retainer 282 is not limited to the rubber balls 282c, and may be O-rings.

Returning to FIG. 8, each of the pins 283 includes a conical convex portion 283a on an outer end in the radial direction, and each of the guides 284 includes a conical concave portion 284a that fits to (engages with) the convex portion 283a on an inner end surface in the radial direction. When the convex portion 283a of the pin 283 fits to the concave portion 284a of the guide 284, the relative rotation position of the retainer 282 with respect to the second shaft 182 is positioned at a predetermined position where the retainer 282 is in the forced free state by a guiding effect of the pins 283 and the guides 284. As shown in FIG. 10, instead of the concave portions 284a of the guides 284, a V-groove 282d may be formed along an axial direction in an inner peripheral portion of the retainer 282. In this way, not only the number of parts and assembly steps can be reduced by eliminating the guides 284, but also axial error of the pins 283 can be allowed.

The operation mechanism 240 includes an operation rod 241 configured to intermittently operate the interrupting units 212 and 222, and a servo motor 242 that linearly moves the operation rod 241.

The second shaft 182 is a hollow shaft having an internal space S2 extending in a rotation axis direction (also referred to as the upper-lower direction), and the operation rod 241 is disposed in the internal space S2. The operation rod 241 is provided with a rack 241a at a lower end exposed from the internal space S2. The operation rod 241 is supported by bearings B4 and B5 disposed in the internal space S2 in a manner of being not relatively rotatable with respect to the rack 241a and being capable of integrally advancing and retracting with the rack 241a in the rotation axis direction. A lid member 188 having an insertion hole through which the operation rod 241 is inserted is screwed to a lower end of the second shaft 182. The lid member 188 prevents foreign matters from entering the internal space S2 and facilitates replacement of the operation rod 241. A pinion 243 provided on an output shaft 242a of the servo motor 242 meshes with the rack 241a, and a position of the operation rod 241 in the upper-lower direction is switched according to the drive of the servo motor 242. The servo motor 242 and the pinion 243 of the present embodiment constitute a drive unit of the present invention. The operation rod 241 is mechanically connected to the drive unit through the rack 241a.

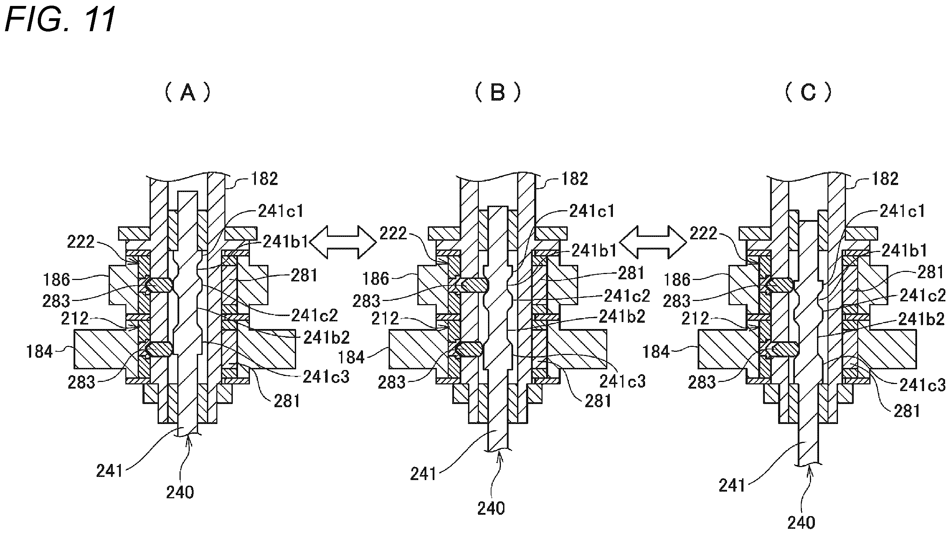

As shown in FIG. 11, the operation rod 241 includes, in the order from the upper side, a first large-diameter portion 241c1, a first small-diameter portion 241b1, a second large-diameter portion 241c2, a second small-diameter portion 241b2, and a third large-diameter portion 241c3 formed with predetermined lengths and intervals therebetween. The operation rod 241 is configured to simultaneously control the two interrupting units 212 and 222, but may be provided separately for each of the interrupting units 212 and 222. Although the operation rod 241 of the present embodiment is integrally formed from the beginning, the operation rod 241 may be formed separately for each of the interrupting units 212 and 222 and then coupled (integrated) thereto. Although the operation rod 241 of the present embodiment changes the positions of the rollers 281 through the pins 283, the guides 284, and the retainer 282, a modification in which the positions of the rollers 281 are changed directly by the pins 283 without using the guides 284 and the retainer 282 can also be applied.

In the following description, operation of the operation mechanism 240 that simultaneously controls the interrupting units 212 and 222 will be described with reference to FIG. 11.

As shown in FIG. 11, the interrupting unit 212 and 222 are switched between the forced free state (hereinafter referred to as an off state as appropriate) and the forced free release state (hereinafter referred to as an on state as appropriate) by the operation mechanism 240.

When the operation rod 241 of the operation mechanism 240 is in an upper position shown in (A) of FIG. 11, the third large-diameter portion 241c3 pushes out the pin 283 of the first interrupting unit 212 in an outer diameter direction while the second large-diameter portion 241c2 pushes out the pin 283 of the second interrupting unit 222 in the outer diameter direction, so that the first interrupting unit 212 and the second interrupting unit 222 are in the off state.

When the operation rod 241 of the operation mechanism 240 is in a middle position shown in (B) of FIG. 11, the third large-diameter portion 241c3 pushes out the pin 283 of the first interrupting unit 212 in the outer diameter direction while the first small-diameter portion 241b1 allows the pin 283 of the second interrupting unit 222 to return in an inner diameter direction, so that the second interrupting unit 222 is in the on state and the first interrupting unit 212 is in the off state.

When the operation rod 241 of the operation mechanism 240 is in a lower position shown in (C) of FIG. 11, the second small-diameter portion 241b2 allows the pin 283 of the first interrupting unit 212 to return in the inner diameter direction while the first large-diameter portion 241c1 pushes out the pin 283 of the second interrupting unit 222 in the outer diameter direction, so that the second interrupting unit 222 is in the off state and the first interrupting unit 212 is in the on state.

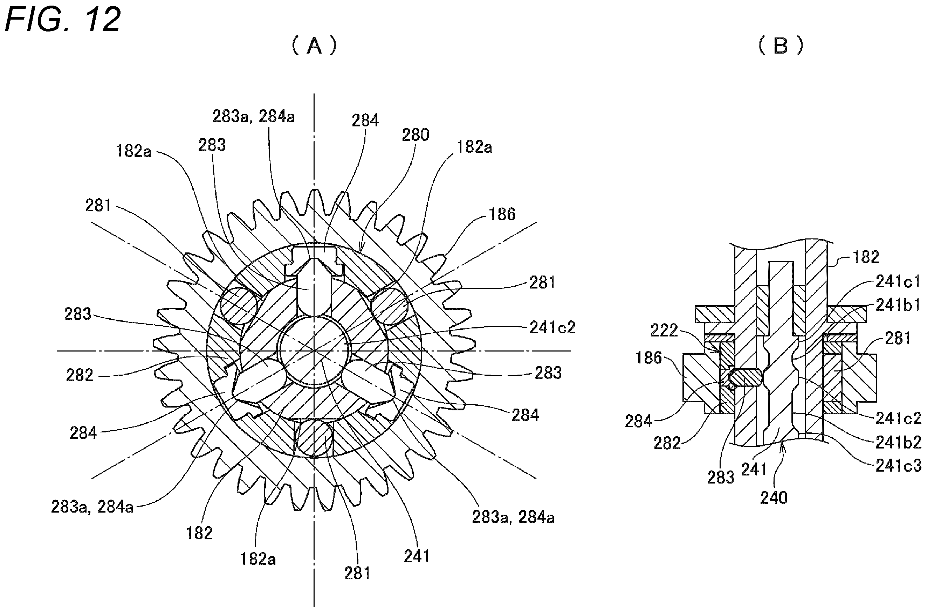

Next, the operation of the two-way clutch 280 will be described with reference to FIGS. 12 to 16, taking the second interrupting unit 222 as an example. In the following example, the case of transition from (A) to (B) to (C) in FIG. 11 in the second interrupting unit 222 will be described as an example.

As shown in (A) and (B) of FIG. 12, when the second large-diameter portion 241c2 of the operation rod 241 pushes out the pin 283 of the second interrupting unit 222 in the outer diameter direction, the convex portion 283a of the pin 283 fits to the concave portion 284a of the guide 284, and the relative rotation position of the retainer 282 with respect to the second shaft 182 is fixed at a predetermined position. In this state, since the rollers 281 are held at the center portion of the flat portions 182a in the circumferential direction, the rollers 281 do not mesh with the outer peripheral surface of the second shaft 182 and the inner peripheral surface of the second driven gear 186, and the state becomes the off state in which the relative rotation between the second shaft 182 and the second driven gear 186 is allowed.

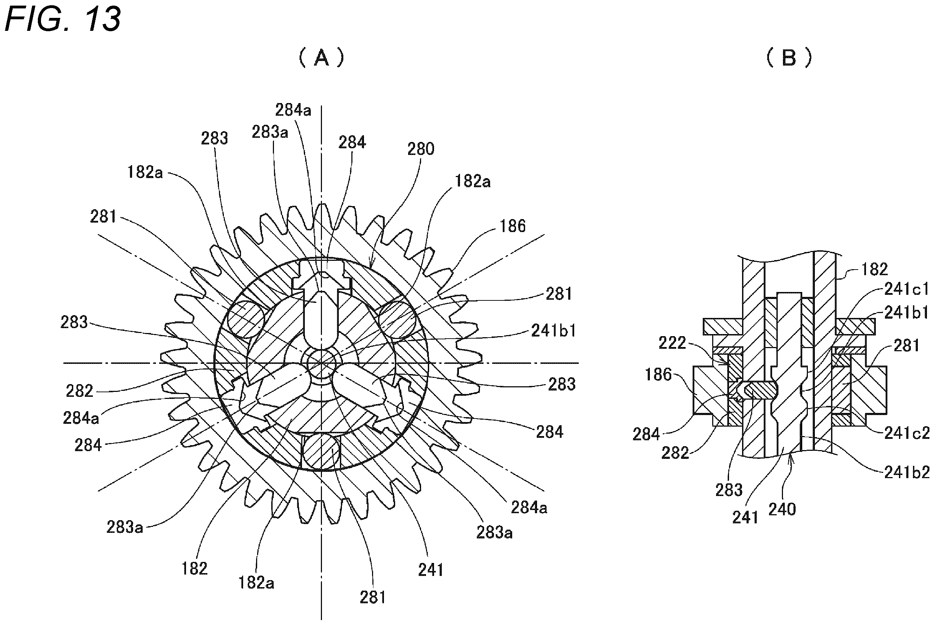

In FIG. 13, (A) and (B) show a state in which the operation rod 241 moves from the position where the second large-diameter portion 241c2 pushes out the pin 283 of the second interrupting unit 222 in the outer diameter direction to the position where the first small-diameter portion 241b1 allows the pin 283 to return in the inner diameter direction. In FIG. 13, the pin 283 is moved in the inner diameter direction, but actually, at the timing when the relative rotation between the second shaft 182 and the second driven gear 186 occurs, the guides 284 of the retainer 282 that rotate together with the second driven gear 186 pushes the pin 283 back in the inner diameter direction on an inclined surface of the concave portion 284a.

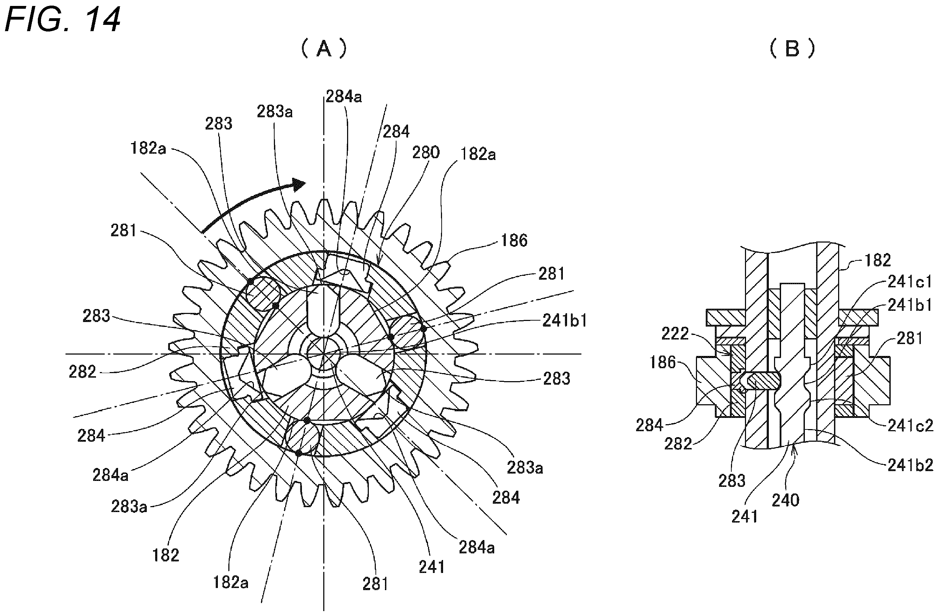

As shown in (A) and (B) of FIG. 14, in a state in which the pin 283 is allowed to return in the inner diameter direction, when relative rotation occurs between the second shaft 182 and the second driven gear 186 in a normal rotation direction shown by the arrow in the drawing, the retainer 282 that rotates together with the second driven gear 186 moves the rollers 281 in the normal rotation direction with respect to the second shaft 182. As a result, the rollers 281 mesh with the outer peripheral surface of the second shaft 182 and the inner peripheral surface of the second driven gear 186, and a normal rotation on state is created in which the second shaft 182 and the second driven gear 186 are rotated integrally in the normal rotation direction.

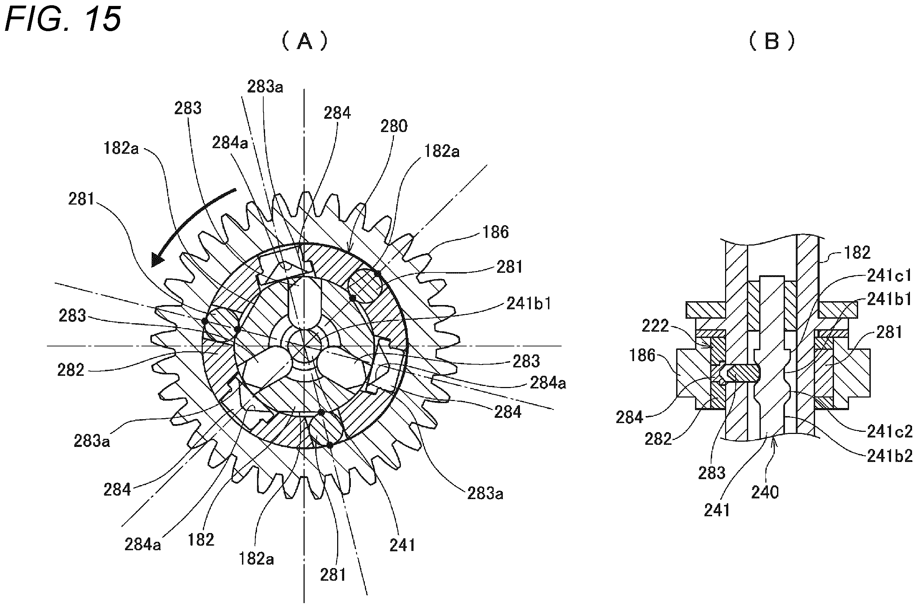

As shown in (A) and (B) of FIG. 15, in a state in which the pin 283 is allowed to return in the inner diameter direction, when relative rotation occurs between the second shaft 182 and the second driven gear 186 in a reverse rotation direction shown by the arrow in the drawing, the retainer 282 that rotates together with the second driven gear 186 moves the rollers 281 in the reverse rotation direction with respect to the second shaft 182. As a result, the rollers 281 mesh with the outer peripheral surface of the second shaft 182 and the inner peripheral surface of the second driven gear 186, and a reverse rotation on state is created in which the second shaft 182 and the second driven gear 186 are rotated integrally in the reverse rotation direction. The retainer 282 can be regarded as an element of an actuator of an operation unit that moves the rollers 281.

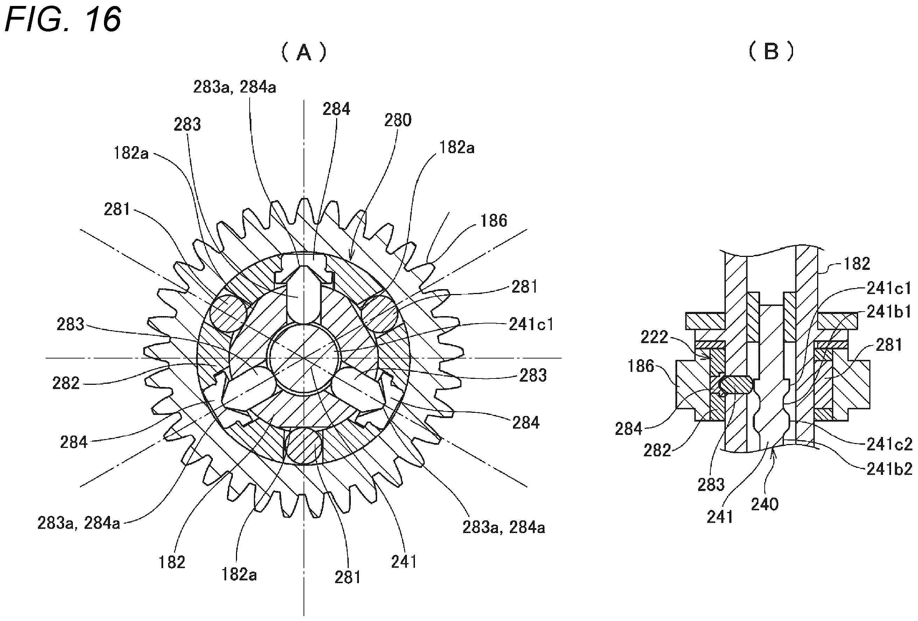

As shown in (A) and (B) of FIG. 16, when the operation rod 241 moves from a position where the first small-diameter portion 241b1 allows the pin 283 of the second interrupting unit 222 to return in the inner diameter direction, to a position where the first large-diameter portion 241c1 pushes out the pin 283 in the outer diameter direction, the convex portion 283a of the pin 283 fits to the concave portion 284a of the guide 284, and due to the guiding effect of the pin 283 and the guide 284, the relative rotation position of the retainer 282 with respect to the second shaft 182 is fixed at a predetermined position. In this state, since the rollers 281 are held at the center portion of the flat portions 182a in the circumferential direction, the rollers 281 do not mesh with the outer peripheral surface of the second shaft 182 and the inner peripheral surface of the second driven gear 186, and the state becomes the off state in which the relative rotation between the second shaft 182 and the second driven gear 186 is allowed.

In the electric prosthetic leg 1 configured in this way, it is possible to smoothly perform a stair ascending operation, which has been required to be done one by one by a leg on a non-prosthetic leg side, with a passive prosthetic leg including a passive damper in the related art.

Specifically, as shown in (A) to (D) of FIG. 17, large power is required when the knee joint mechanism 130 is stretched from a bending state, in a state in which a load is applied to the electric prosthetic leg 1 when moving the electric prosthetic leg 1 forward to ascending stairs.

In this case, the transmission T is set to a transmission state in which the operation rod 241 is positioned at the position shown in (C) of FIG. 11. In this transmission state, the motor M and the spindle unit SP are in a power transmission state through the first transmission mechanism T1. In this state, when the motor M is rotated in a first direction, the power of the motor M is transmitted to the first shaft 181, the first drive gear 183, the first driven gear 184, the interrupting unit 212 of the first interrupting mechanism 210, the second shaft 182, and the spindle unit SP. Accordingly, the sleeve 174 is translated (elongated) so as to be separated from the transmission T, and the above-knee member 120 to which the sleeve 174 is coupled is rotated about the pivoting portion 135 with respect to the below-knee member 110 to which the transmission T is attached, and the knee joint mechanism 130 is stretched. Since the power for this stretching is power whose torque is increased during deceleration by the first transmission mechanism T1, even when a large load is applied to the electric prosthetic leg 1 when moving the electric prosthetic leg 1 forward to ascending stairs, it is also possible to reliably stretch the knee joint mechanism 130 from the bending state.

On the other hand, in order to smoothly ascend stairs, as shown in (E) to (H) of FIG. 17, it is necessary to bend (lift) the knee joint mechanism 130 from the stretching state while a load is applied to the healthy leg. When the knee joint mechanism 130 is bent from the stretching state, large power is not required, but quick action is required.

In this case, the transmission T is set to a transmission state in which the operation rod 241 is positioned at the position shown in (B) of FIG. 11. In this transmission state, the motor M and the spindle unit SP are in a power transmission state through the second transmission mechanism T2. In this state, when the motor M is rotated in a second direction which is opposite to the first direction, the power of the motor M is transmitted to the first shaft 181, the second drive gear 185, the second driven gear 186, the interrupting unit 222 of the second interrupting mechanism 220, the second shaft 182, and the spindle unit SP. Accordingly, the sleeve 174 is translated (shortened) so as to approach the transmission T, and the below-knee member 110 to which the transmission T is attached rotate about the pivoting portion 135 with respect to the above-knee member 120 to which the sleeve 174 is attached, and the knee joint mechanism 130 is bent. Since the power for this bending is power whose torque is reduced in acceleration by the second transmission mechanism T2, it becomes possible to quickly bend the knee joint mechanism 130.

During walking on level ground and during descending of stairs (stair descending) shown in FIG. 18, the transmission T is set to the transmission state in which the operation rod 241 is positioned at the position shown in (A) of FIG. 11 when the electric prosthetic leg 1 is an idling leg to which no load is applied. In this transmission state, since the first interrupting unit 212 and the second interrupting unit 222 are in the off state, the motor M and the spindle unit SP are in a free state of being disconnected. In this state, any kind of stretching and bending of the knee joint mechanism 130 are allowed according to a walking situation, so that smooth walking with the prosthetic leg as an idling leg can be performed.

On the other hand, during walking on level ground and during descending of stairs (stair descending) shown in FIG. 18, the transmission T is set to the transmission state in which the operation rod 241 is positioned at the position shown in (B) of FIG. 11 when the electric prosthetic leg 1 is a standing leg to which a load is applied. In this transmission state, since the second interrupting unit 222 is in the on state, the motor M and the spindle unit SP are in a power transmission state through the second transmission mechanism T2. In this state, since an external force acting on the electric prosthetic leg 1 in a bending direction is transmitted from the spindle unit SP to the motor M through the second transmission mechanism T2, the friction of the motor M is used to attenuate the external force in the bending direction, and therefore smooth walking with the prosthetic leg as a standing leg can be achieved.

According to the present embodiment, when the operation rod 241 is moved in the upper-lower direction to switch between the position shown in (A) of FIG. 11 and the position shown in (B) of FIG. 11, it is possible to quickly switch the transmission state during descending of stairs (stair descending) and during walking on level ground without going through the position shown in (C) of FIG. 11.

During walking on level ground and during descending of stairs (stair descending) shown in FIG. 18, the transmission T may be set to a transmission state in which the operation rod 241 is at the position of (C) in FIG. 11. In this case, the external force in the bending direction acting on the electric prosthetic leg 1 is transmitted from the spindle unit SP to the motor M through the first transmission mechanism T1. Whether the external force in the bending direction is attenuated through the first transmission mechanism T1 or the external force in the bending direction is attenuated through the second transmission mechanism T2 can be appropriately changed according to the physique or preference of the user of the electric prosthetic leg 1. In the case where the external force in the bending direction is attenuated through the first transmission mechanism T1, when the operation rod 241 is moved in the upper-lower direction, it is preferable that the position shown in (B) of FIG. 11 does not enter between the position shown in (A) of FIG. 11 and the position shown in (C) of FIG. 11. In this case, it is necessary to replace with an operation rod 241 with different positions and numbers of small-diameter portions 241b1, 241b2 and large-diameter portions 241c1 to 241c3. As described above, since the lid member 188 having the insertion hole through which the operation rod 241 is inserted is screwed to the lower end of the second shaft 182, the operation rod 241 can be easily replaced by removing the lid member 188.

### (First Modification)

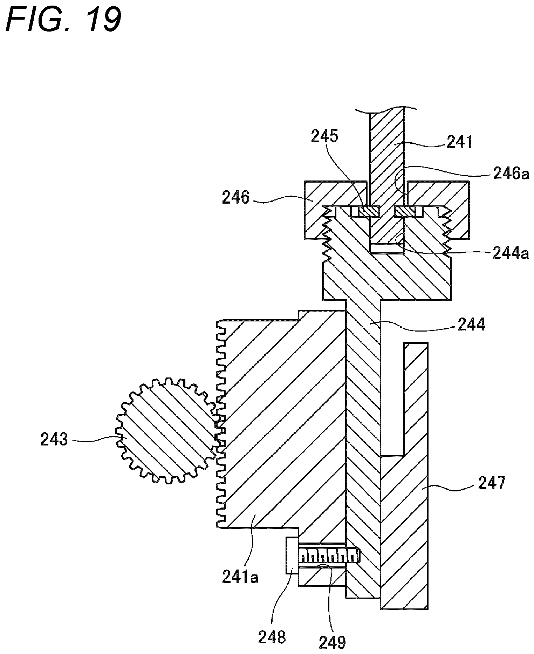

FIG. 19 is a cross-sectional view showing a coupling portion between the operation rod 241 and the rack 241a in the electric prosthetic leg 1 of a first modification.

In the embodiment described above, the operation rod 241 is configured to be unable to rotate relative to the rack 241a and to be able to advance and retract integrally with the rack 241a in the rotation axis direction, but in the first modification, the operation rod 241 is configured to be able to rotate relative to the rack 241a and to advance and retract integrally with the rack 241a in the rotation axis direction. In other words, the operation rod 241 is provided to be rotatable with respect to the rotation axis and to be capable of advancing and retracting in the rotation axis direction, and the rack 241a is provided to be non-rotatable with respect to the rotation axis and to be capable of advancing and retracting in the rotation axis direction.

In the first modification, the rack 241a is fixed to a rack gear holder 244, and the operation rod 241 is supported by a hole 244a provided in an upper surface of the rack gear holder 244. More specifically, an E-ring (snap ring) 245 is fitted to the lower end of the operation rod 241 in a manner of being not movable in the rotation axis direction with respect to the operation rod 241, and a rod holder nut 246 provided with an insertion hole 246a through which the operation rod 241 is inserted is screwed to the upper end of the operation rod 241 so as to sandwich the E-ring 245. The rack gear holder 244 is supported on a slider base 247 in a manner of being slidable in the rotation axis direction.

Accordingly, when the pinion 243 provided on the output shaft 242a of the servo motor 242 rotates and the rack 241a moves in the upper-lower direction, the rack gear holder 244, the rod holder nut 246, the E-ring 245, and the operation rod 241 move in the upper-lower direction together with the rack 241a. On the other hand, since the operation rod 241 is rotatable relative to the E-ring 245, the operation rod 241 is rotatable relative to the rack gear holder 244.

Accordingly, when the operation rod 241 moves from the position where the first small-diameter portion 241b1 allows the pin 283 of the second interrupting unit 222 to return in the inner diameter direction to the position where the first large-diameter portion 241c1 pushes out the pin 283 in the outer diameter direction as described in (A) and (B) of FIG. 16, the force received by the pin 283 and the guide 284 becomes the rotation force of the operation rod 241, and the operation load of the operation rod 241 can be reduced.

The rack 241a is fixed to the rack gear holder 244 by the screw 248, and the rack 241a is preferably provided with a slot hole 249 whose fastening position can be adjusted. Accordingly, since the relative positions of the pinion 243 and the rack 241a can be adjusted, the relative positions of the pinion 243 and the operation rod 241 can be easily determined during assembly.

### (Second Modification)

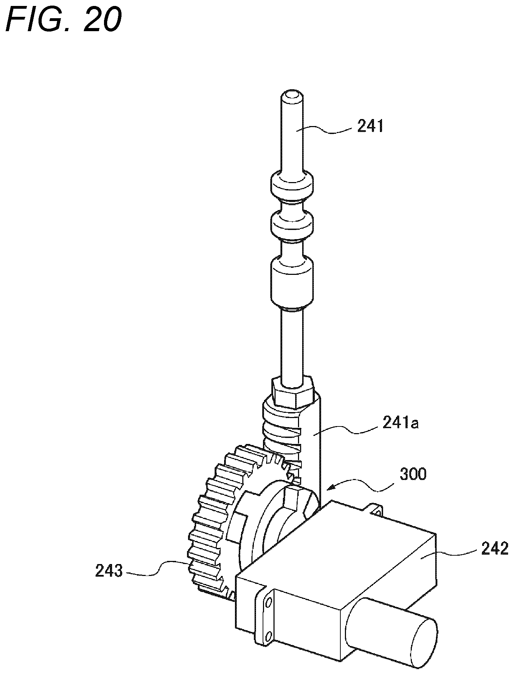

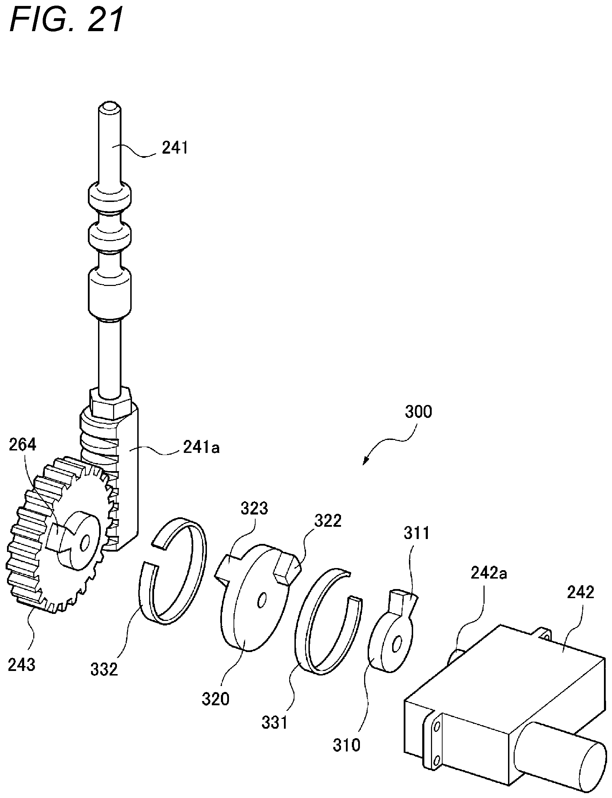

FIG. 20 is a perspective view of the drive unit of the electric prosthetic leg 1 of the second modification, and FIG. 21 is an exploded perspective view of the drive unit of the electric prosthetic leg 1 of the second modification.

The drive unit of the electric prosthetic leg 1 of the second modification includes the servo motor 242, the pinion 243 that meshes with the rack 241a provided on the operation rod 241, and an elastic energy accumulating mechanism 300 provided between the servo motor 242 and the pinion 243. The elastic energy accumulating mechanism 300 includes a first rotation bracket 310 provided on the output shaft 242a of the servo motor 242, a second rotation bracket 320 disposed between the first rotation bracket 310 and the pinion 243, and a C-shaped first elastic member 331 and a C-shaped second elastic member 332.

The first rotation bracket 310 is provided with a first engagement portion 311 protruding in the radial direction, and the first engagement portion 311 is engaged in the circumferential direction with a second engagement portion 322 protruding from a surface of the second rotation bracket 320 on the first rotation bracket 310 side toward the first rotation bracket 310 side. The second rotation bracket 320 is provided with a third engagement portion 323 that protrudes from a surface on the pinion 243 side to the pinion 243 side, and the third engagement portion 323 is engaged in the circumferential direction with a fourth engagement portion 264 protruding from a surface of the pinion 243 on the second rotation bracket 320 side toward the second rotation bracket 320 side.

The direction in which the first engagement portion 311 of the first rotation bracket 310 engages with the second engagement portion 322 of the second rotation bracket 320 is the same as the direction in which the fourth engagement portion 264 of the pinion 243 engages with the third engagement portion 323 of the second rotation bracket 320. The first elastic member 331 biases the first engagement portion 311 and the second engagement portion 322 in the engagement direction, and the second elastic member 332 biases the third engagement portion 323 and the fourth engagement portion 264 in the engagement direction.

Accordingly, the rotation toward one side of the servo motor 242 (hereinafter, referred to as a normal rotation direction) is input from the first engagement portion 311 of the first rotation bracket 310 to the second engagement portion 322 of the second rotation bracket 320 through the first elastic member 331, and is directly transmitted from the third engagement portion 323 of the second rotation bracket 320 to the fourth engagement portion 264 of the pinion 243. On the other hand, the rotation toward the other side of the servo motor 242 (hereinafter, referred to as a reverse rotation direction) is directly input from the first engagement portion 311 of the first rotation bracket 310 to the second engagement portion 322 of the second rotation bracket 320, and is transmitted from the third engagement portion 323 of the second rotation bracket 320 to the fourth engagement portion 264 of the pinion 243 through the second elastic member 332.

Next, functions of the elastic energy accumulating mechanism 300 configured as described above will be described.

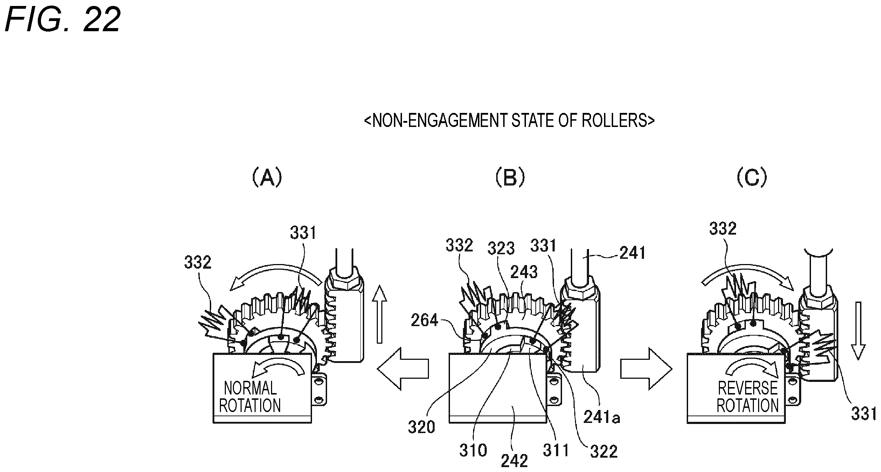

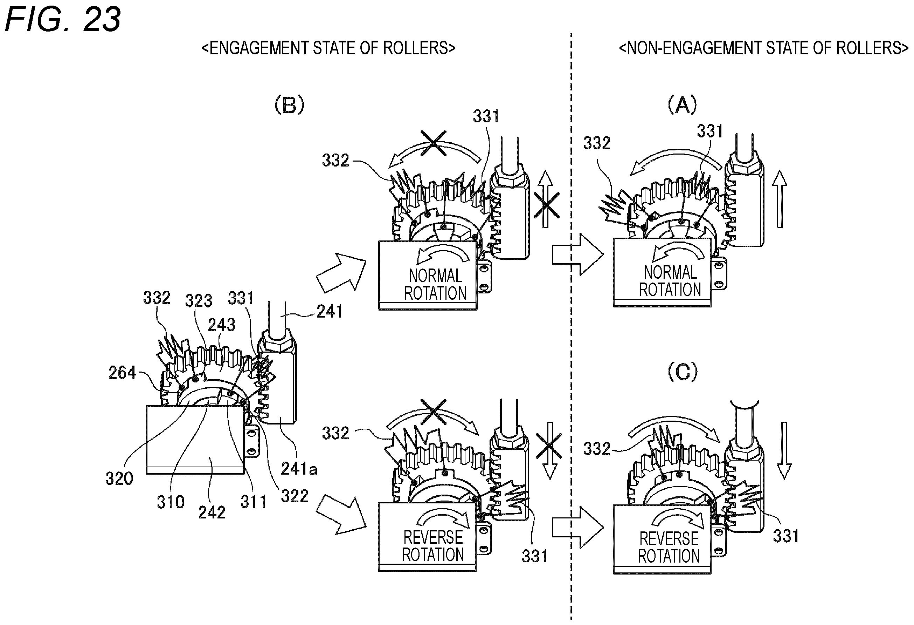

FIGS. 22 and 23 are diagrams illustrating the operation of the drive unit in the electric prosthetic leg 1 of the second modification. The position shown in FIG. 22 and (A) of FIG. 23 is the position shown in (A) of FIG. 11, and the position shown in FIG. 22 and (B) of FIG. 23 is the position shown in (B) of FIG. 11, and the position shown in FIG. 22 and (C) of FIG. 23 is the position shown in (C) of FIG. 11. In FIGS. 22 and 23, the first elastic member 331 and the second elastic member 332 are schematically illustrated, and a state in which the first elastic member 331 and the second elastic member 332 extend in the circumferential direction indicates a state in which elastic energy is accumulated.

In the non-engagement state of the rollers 281, the movement of the operation rod 241 in the upper-lower direction is not limited. Therefore, when the servo motor 242 is rotated in the normal rotation direction from the position shown in (B) of FIG. 22, the operation rod 241 moves to the position shown in (A) of FIG. 22 (the position shown in (A) of FIG. 11). In this case, since the movement of the operation rod 241 in the upper-lower direction is not limited, elastic energy is not accumulated in the first elastic member 331.

When the servo motor 242 is rotated in the reverse rotation direction from the position shown in (B) of FIG. 22, the operation rod 241 moves to the position shown in (C) of FIG. 22 (the position shown in (C) of FIG. 11). In this case, since the movement of the operation rod 241 in the upper-lower direction is not limited, elastic energy is not accumulated in the second elastic member 332.

In the engagement state of the rollers 281, the movement of the operation rod 241 in the upper-lower direction is limited. Therefore, even when the servo motor 242 is rotated in the normal rotation direction from the position shown in (B) of FIG. 22 (the state shown in the left side of FIG. 23), the operation rod 241 does not move, and the pinion 243 and the second rotation bracket 320 do not rotate accordingly. Accordingly, the first engagement portion 311 of the first rotation bracket 310 rotates in the normal rotation direction with respect to the second engagement portion 322 of the second rotation bracket 320, and the elastic energy is accumulated in the first elastic member 331 (the state shown in an upper side in the middle of FIG. 23). When the rollers 281 transition from the engagement state to the non-engagement state, the elastic energy of the first elastic member 331 makes the second rotation bracket 320 and the pinion 243 rotate in the normal rotation direction, and the operation rod 241 moves to the position shown in (A) of FIG. 22 (the position shown in (A) of FIG. 11) (the state shown in the upper right side of FIG. 23).

Even when the servo motor 242 is rotated in the reverse rotation direction from the position shown in (B) of FIG. 22 (the state shown in the left side of FIG. 23), the operation rod 241 does not move, and the pinion 243 does not rotate accordingly. Accordingly, the third engagement portion 323 of the second rotation bracket 320 rotates in the reverse rotation direction with respect to the fourth engagement portion 264 of the pinion 243, and elastic energy is accumulated in the second elastic member 332 (the state shown in a lower side in the middle of FIG. 23). When the rollers 281 transition from the engagement state to the non-engagement state, the elastic energy of the second elastic member 332 makes the pinion 243 rotate in the reverse rotation direction, and the operation rod 241 moves to the position shown in (C) of FIG. 22 (the position shown in (C) of FIG. 11) (the state shown in the lower right side of FIG. 23).

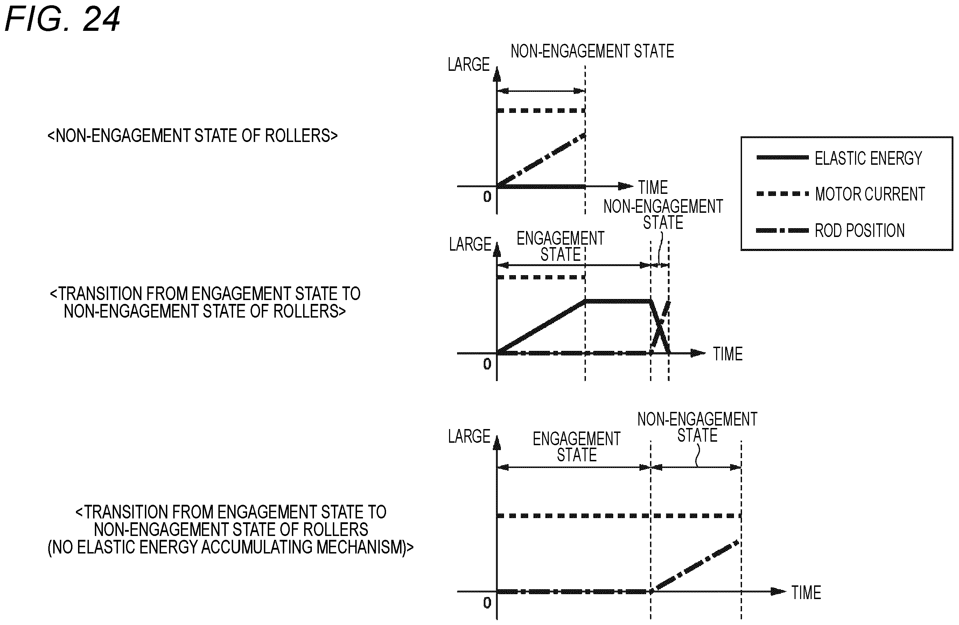

FIG. 24 is a graph showing a current value of the servo motor 242, a position of the operation rod 241, and elastic energy of the elastic members 331 and 332 in the case of the non-engagement state of the rollers 281, transition from the engagement state to the non-engagement state of the rollers 281, and transition from the engagement state to the non-engagement state of the rollers 281 (without elastic energy accumulating mechanism). The vertical axis represents the current value of the servo motor 242, the position of the operation rod 241, and the elastic energy of the elastic members 331 and 332, and the horizontal axis represents time.

As shown in the upper graph of FIG. 24, in the non-engagement state of the rollers 281, the operation rod 241 moves according to the current of the servo motor 242. On the other hand, when the operation rod 241 is moved by applying a current to the servo motor 242 from the engagement state of the rollers 281, the movement of the operation rod 241 in the upper-lower direction is limited as described above. In this case, if the elastic energy accumulating mechanism 300 is not provided, as shown in the lower graph of FIG. 24, the servo motor 242 continues to apply current until the rollers 281 change from the engagement state to the non-engagement state and the operation rod 241 moves to a predetermined position. Therefore, an excessive load may be applied to the servo motor 242 and the servo motor 242 may be damaged.

On the other hand, in the case of the present modification including the elastic energy accumulating mechanism 300, as shown in the middle graph of FIG. 24, the servo motor 242 stops the current at the time when predetermined elastic energy is accumulated in the elastic energy accumulating mechanism 300. Therefore, no excessive load is applied to the servo motor 242. The operation rod 241 moves to a predetermined position when the rollers 281 transition from the engagement state to the non-engagement state due to the elastic energy of the first elastic member 331 or the second elastic member 332 accumulated in the elastic energy accumulating mechanism 300. In this way, according to the second modification, it is possible to reduce power consumption of the servo motor 242 and to prevent damage to the servo motor 242 due to an excessive load.

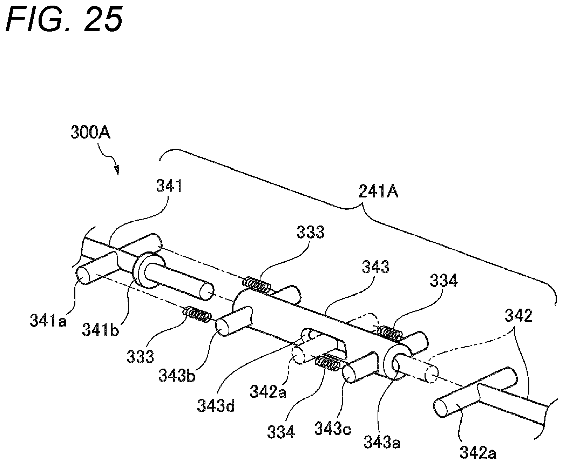

FIG. 25 is a diagram showing another example of the elastic energy accumulating mechanism 300 (hereinafter referred to as an elastic energy accumulating mechanism 300A).

The elastic energy accumulating mechanism 300A includes a third elastic member 333 and a fourth elastic member 334 which are tension coil springs. Note that the operation rod 241 used in the elastic energy accumulating mechanism 300A is divided into at least three parts, and the operation rod 241 is hereinafter referred to as an operation rod 241A. Note that as another example, the operation rod 241A may be a part of the operation rod 241 of the above embodiment, or may be a separate member attached to the operation rod 241.

The operation rod 241A includes a first operation rod 341 provided with small-diameter portions 241b1 and 241b2 and large-diameter portions 241c1 to 241c3 (not shown in FIG. 25), a second operation rod 342 provided with a rack 241a (not shown in FIG. 25), and a hollow third operation rod 343 provided between the first operation rod 341 and the second operation rod 342, in which the first operation rod 341 to the third operation rod 343 are assembled so as to be relatively movable in an axial direction.

The first operation rod 341 includes a first spring shoe 341a and a position regulating portion 341b in order from the upper side, and is configured such that a portion below the position regulating portion 341b is inserted into a hollow portion 343a of the third operation rod 343. A second spring shoe 343b is provided at an upper end of the third operation rod 343, and the third elastic member 333 is disposed between the first spring shoe 341a of the first operation rod 341 and the second spring shoe 343b.

In the third operation rod 343, a pair of guide slots 343d in communication with the hollow portion 343a is formed below the second spring shoe 343b.

A third spring shoe 342a is provided at an upper end of the second operation rod 342. An upper portion of the second operation rod 342 is inserted into the hollow portion 343a of the third operation rod 343, and the third spring shoe 342a is exposed to the outside from the pair of guide slots 343d.

A fourth spring shoe 343c is provided at a lower end of the third operation rod 343, and the fourth elastic member 334 is disposed between the third spring shoe 342a of the second operation rod 342 exposed to the outside from the pair of guide slots 343d and the fourth spring shoe 343c.

Functions of the elastic energy accumulating mechanism 300A configured as described above will be described.

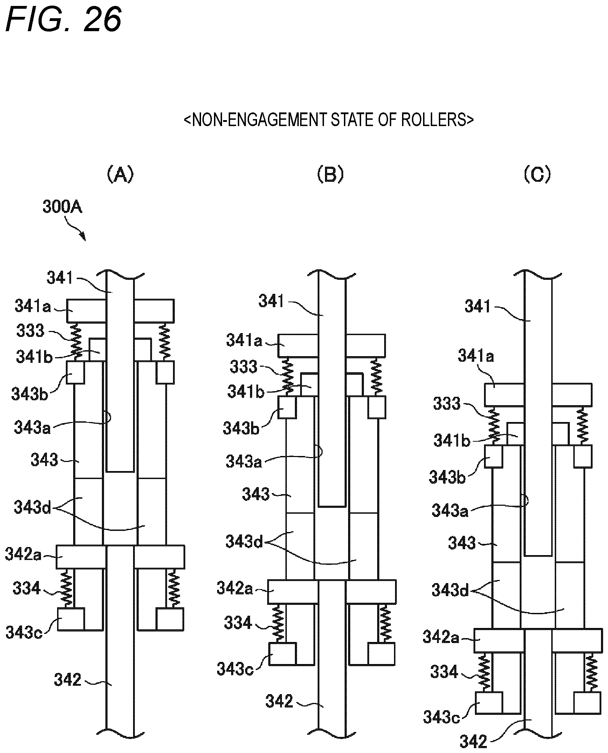

FIG. 26 is a diagram illustrating operation of the elastic energy accumulating mechanism 300A. The position shown in (A) of FIG. 26 is the position shown in (A) of FIG. 11, and the position shown in (B) of FIG. 26 is the position shown in (B) of FIG. 11, and the position shown in (C) of FIG. 26 is the position shown in (C) of FIG. 11.

In the non-engagement state of the rollers 281, the movement of the operation rod 241A in the upper-lower direction is not limited. Therefore, when the servo motor 242 is rotated in the normal rotation direction from the position shown in (B) of FIG. 26, the operation rod 241A moves to the position shown in (A) of FIG. 26 (the position shown in (A) of FIG. 11). That is, when the second operation rod 342 provided with the rack 241a moves to the upper side, the third operation rod 343 moves to the upper side through the fourth elastic member 334, and when the third operation rod 343 moves to the upper side, the first operation rod 341 moves to the upper side through the third elastic member 333. In this case, since the movement of the operation rod 241A in the upper-lower direction is not limited, elastic energy is not accumulated in the third elastic member 333 and the fourth elastic member 334.

When the servo motor 242 is rotated in the reverse rotation direction from the position shown in (B) of FIG. 26, the operation rod 241A moves to the position shown in (C) of FIG. 26 (the position shown in (C) of FIG. 11). That is, when the second operation rod 342 provided with the rack 241a moves to the lower side, the third operation rod 343 moves to the lower side through the fourth elastic member 334, and when the third operation rod 343 moves to the lower side, the first operation rod 341 moves to the lower side through the third elastic member 333. In this case, since the movement of the operation rod 241A in the upper-lower direction is not limited, elastic energy is not accumulated in the third elastic member 333 and the fourth elastic member 334.

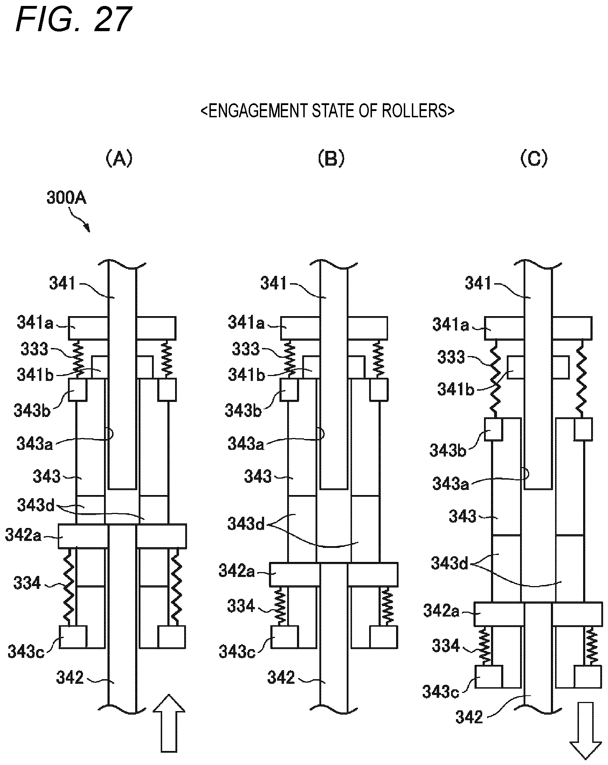

In the engagement state of the rollers 281, the movement of the operation rod 241A in the upper-lower direction is limited. Therefore, even when the servo motor 242 is rotated in the normal rotation direction from the position shown in (B) of FIG. 27 (the same position as shown in (B) of FIG. 26), the first operation rod 341 does not move and the third operation rod 343 does not move. Accordingly, only the second operation rod 342 moves to the upper side, and the distance between the spring shoe 342a of the second operation rod 342 and the spring shoe 343c of the third operation rod 343 changes, so that elastic energy is accumulated in the fourth elastic member 334 (the state shown in (A) of FIG. 27). When the rollers 281 transition from the engagement state to the non-engagement state, the elastic energy of the fourth elastic member 334 makes the third operation rod 343 move to the upper side, and accordingly, the first operation rod 341 moves to the position shown in (A) of FIG. 26 (the position shown in (A) of FIG. 11) through the third elastic member 333.

Even when the servo motor 242 is rotated in the reverse rotation direction from the position shown in (B) of FIG. 27 (the same position as shown in (B) of FIG. 26), the first operation rod 341 does not move and the second operation rod 342 moves to the lower side, and accordingly, the third spring shoe 342a comes into contact with lower ends of the guide slots 343d, and the third operation rod 343 is moved to the lower side by the third spring shoe 342a. Therefore, as the distance between the spring shoe 341a of the first operation rod 341 and the spring shoe 343b of the third operation rod 343 changes, elastic energy is accumulated in the third elastic member 333 (the state shown in (C) of FIG. 27). When the rollers 281 transition from the engagement state to the non-engagement state, the elastic energy of the third elastic member 333 makes the first operation rod 341 move to the lower side, so that the first operation rod 341 moves to the position shown in (C) of FIG. 26 (the position shown in (C) of FIG. 11).

### (Third Modification)

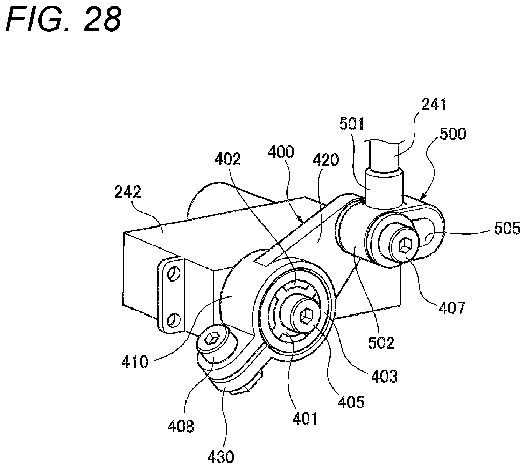

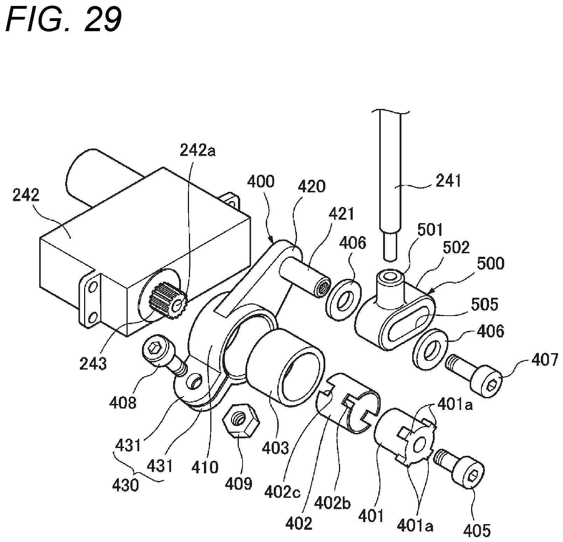

FIG. 28 is a perspective view of the drive unit of the electric prosthetic leg 1 of the third modification, and FIG. 29 is an exploded perspective view of the drive unit of the electric prosthetic leg 1 of the third modification.

In the above-described embodiment, the drive unit of the operation rod 241 is configured by a rack-and-pinion mechanism including the rack 241a and the pinion 243, but in the third modification, a link mechanism is used.

The drive unit of the electric prosthetic leg 1 of the third modification includes the servo motor 242, a driven gear 401, a torsion spring 402, a spacer 403, and an arm portion 400 coupled to a coupling portion 500 provided on the operation rod 241. The coupling portion 500 includes a rod fixing portion 501 fixed to the lower end of the operation rod 241, and an arm coupling portion 502 provided below the rod fixing portion 501. The arm coupling portion 502 is formed with a long hole 505 that penetrates in an output shaft line direction of the servo motor 242 and is elongated in a direction orthogonal to the rotation axis direction (upper-lower direction) of the operation rod 241 and the output shaft line direction of the servo motor 242.

The arm portion 400 includes a cylindrical arm body 410 that accommodates the driven gear 401, the torsion spring 402, and the spacer 403, an arm piece 420 that extends from the arm body 410 and is provided with an engagement pin 421 that is guided by the long hole 505 of the arm coupling portion 502, and an arm fixing portion 430 that is provided on the opposite side of the arm piece 420 with the arm body 410 interposed therebetween. The arm body 410 is partially cut in the circumferential direction, and the arm fixing portion 430 includes a pair of fixing pieces 431 extending from both ends thereof.

The driven gear 401 has a cylindrical shape, and an inner peripheral gear (not shown) meshing with the pinion 243 provided on the output shaft 242a of the servo motor 242 is provided on an inner peripheral portion on the servo motor 242 side, and four teeth 401a are provided on an outer peripheral portion on the opposite side to the servo motor 242. The torsion spring 402 is provided in a cylindrical shape so as to cover an outer peripheral portion of the driven gear 401, is provided with a plurality of engagement grooves 402b that engage with the plurality of teeth 401a on the opposite side to the servo motor 242, and is also provided with a plurality of engagement grooves 402c on the servo motor 242 side. The spacer 403 is provided in a cylindrical shape so as to cover an outer peripheral portion of the torsion spring 402, and a plurality of engagement claws (not shown) engaged with the plurality of engagement grooves 402c of the torsion spring 402 are provided on an inner peripheral portion of the spacer 403 on the servo motor 242 side.

In assembly, the torsion spring 402 is disposed on an inner peripheral portion of the spacer 403 such that the engagement grooves 402c are engaged with the engagement claws of the spacer 403, and the driven gear 401 is disposed on the inner peripheral portion of the torsion spring 402 such that the teeth 401a of the driven gear 401 are engaged with the engagement grooves 402b of the torsion spring 402. In a state where the inner peripheral gear of the driven gear 401 and the pinion 243 of the servo motor 242 are fitted to each other, the bolt 405 is fastened to the output shaft 242a of the servo motor 242. In the arm portion 400, the bolt 407 is fastened to the engagement pin 421 in a state where the engagement pin 421 is inserted into the long hole 505 of the arm coupling portion 502 and a pair of washers 406 is disposed so as to sandwich the arm coupling portion 502. In a state where the spacer 403 is disposed on the inner peripheral portion of the arm body 410 of the arm portion 400, the pair of fixing pieces 431 is fastened by the bolt 408 and the nut 409, so that the spacer 403 is held by the arm body 410.

Next, functions of the drive unit in the electric prosthetic leg 1 of the third modification configured as described above will be described.

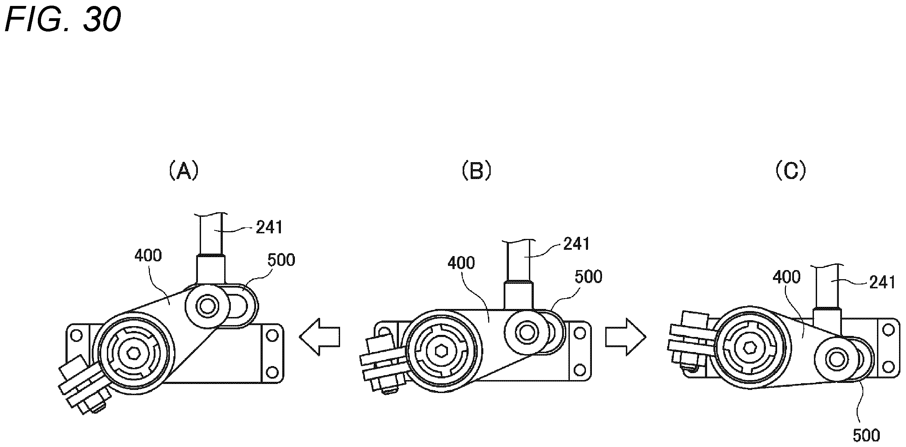

FIG. 30 is a diagram illustrating the operation of the drive unit in the electric prosthetic leg 1 of the third modification. The position shown in (A) of FIG. 30 is the position shown in (A) of FIG. 11, and the position shown in (B) of FIG. 30 is the position shown in (B) of FIG. 11, and the position shown in (C) of FIG. 30 is the position shown in (C) of FIG. 11.

As shown in (A), (B), and (C) of FIG. 30, the engagement pin 421 moves in the long hole 505 according to the position of the operation rod 241 in the rotation axis direction (upper-lower direction). Therefore, when the arm portion 400 rotates together with the rotation of the servo motor 242, the long hole 505 and the engagement pin 421 transmit the movement of the arm portion 400 of the operation rod 241 in the rotation axis direction (upper-lower direction in the drawing) to the coupling portion 500, but do not transmit the arm portion 400 in the direction (the lateral direction in the drawing) orthogonal to the rotation axis direction (upper-lower direction) of the operation rod 241 and the output shaft line direction of the servo motor 242 to the coupling portion 500.

According to the third modification, assembly error can be prevented, and workability is improved. When an overload occurs in the servo motor 242, the torsion spring 402 is twisted to reduce the load on the servo motor 242, thereby preventing the servo motor 242 from being damaged.

### (Fourth Modification)

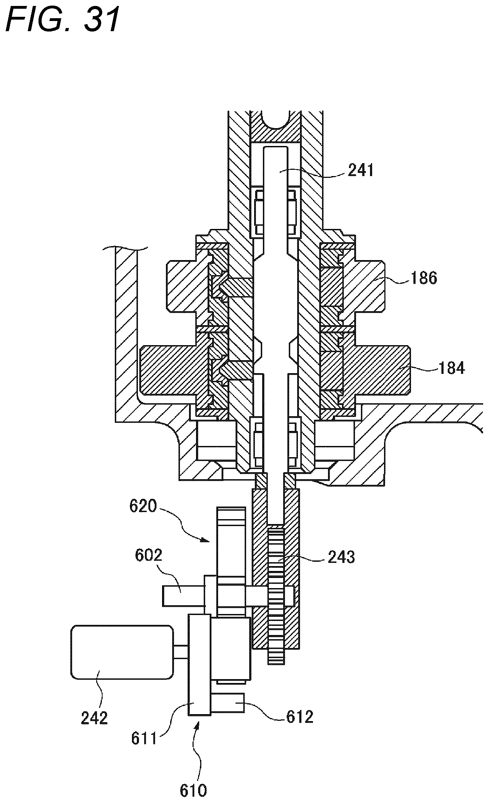

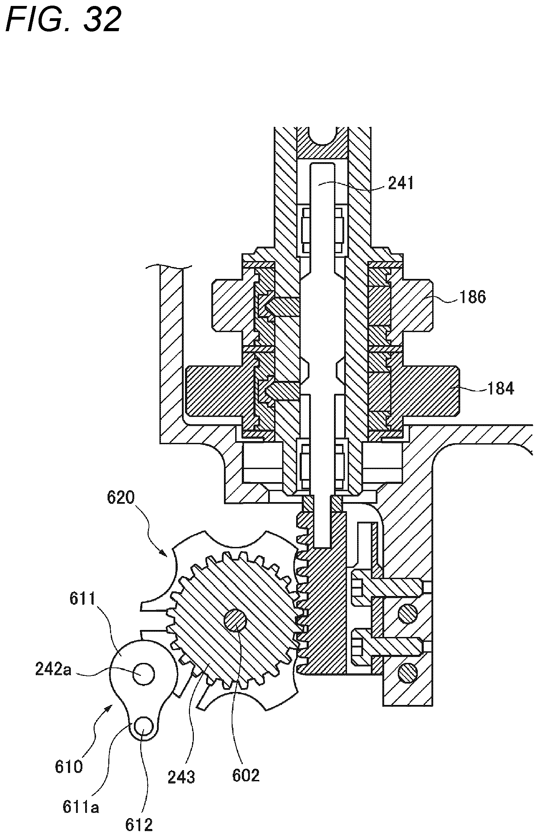

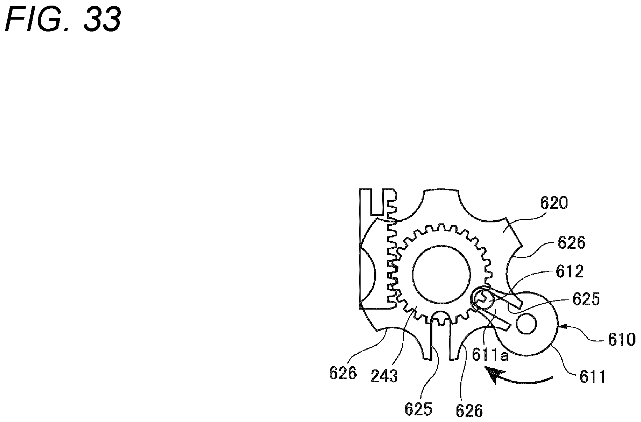

FIG. 31 is a cross-sectional view of the drive unit in the electric prosthetic leg 1 of the fourth modification, and FIG. 32 is another cross-sectional view of the drive unit of the fourth modification, and FIG. 33 is a diagram illustrating the Geneva mechanism.

The drive unit of the electric prosthetic leg 1 of the fourth modification includes the servo motor 242, a Geneva drive gear 610 provided on the output shaft 242a of the servo motor 242, a Geneva driven gear 620 supported by a rotation shaft 602 extending in parallel with the output shaft 242a, and the pinion 243 supported by the rotation shaft 602 and meshing with the rack 241a provided on the operation rod 241.

Referring to FIG. 33, the Geneva drive gear 610 includes an arc portion 611 having an arc shape centered on the output shaft 242a, and a drive pin 612 provided on an extending portion 611a extending from an outer side in the radial direction from the arc portion 611 and protruding toward the Geneva driven gear 620. The Geneva driven gear 620 is formed with two substantially U-shaped slots 625 which are spaced apart from each other by 60° centered on the rotation shaft 602 and extend to the outer side in the radial direction, and concave arc portions 626 which are formed between the two slots 625 and on both sides of the two slots in the circumferential direction and are concave toward the rotation shaft 602. The concave arc portions 626 are set to have the same curvature as the curvature of the arc portion 611 of the Geneva drive gear 610, and are guided by the arc portion 611 of the Geneva drive gear 610.

As shown in FIG. 33, when the Geneva drive gear 610 rotates, the drive pin 612 of the Geneva drive gear 610 engages with the slots 625 of the Geneva driven gear 620 and rotates while sliding in the slots 625, and when the Geneva drive gear 610 rotates by one cycle, the Geneva driven gear 620 is rotated by 60°. After the Geneva driven gear 620 is rotated by 60° and the drive pin 612 is separated from the slots 625, the Geneva drive gear 610 continuously rotates and the Geneva driven gear 620 stops. That is, the Geneva drive gear 610 and the Geneva driven gear 620 convert the continuous rotation input to the Geneva drive gear 610 into intermittent rotation and output the intermittent rotation from the Geneva driven gear 620.

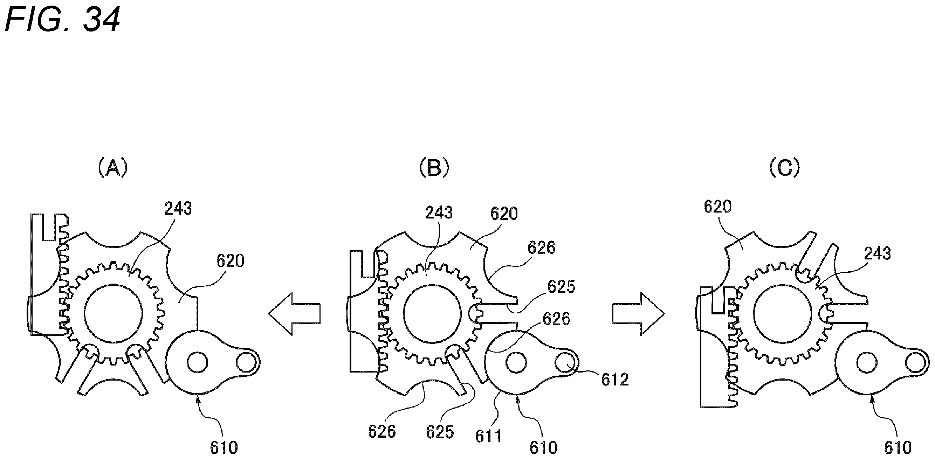

FIG. 34 is a diagram illustrating the operation of the drive unit in the electric prosthetic leg 1 of the fourth modification. The position shown in (A) of FIG. 34 is the position shown in (A) of FIG. 11, and the position shown in (B) of FIG. 34 is the position shown in (B) of FIG. 11, and the position shown in (C) of FIG. 34 is the position shown in (C) of FIG. 11.

According to the fourth modification, since the position of the operation rod 241 is determined by the servo motor 242 making the Geneva drive gear 610 rotate for one cycle, it is not necessary to perform position control by constantly energizing the servo motor 242, and the power consumption of the servo motor 242 can be reduced, and the control can be simplified. Since the Geneva mechanism includes a mechanical stop mechanism, the rotation from the Geneva driven gear 620 is not transmitted to the Geneva drive gear 610, and the position of the operation rod 241 is not shifted even when an external force is applied.

### (Fifth Modification)

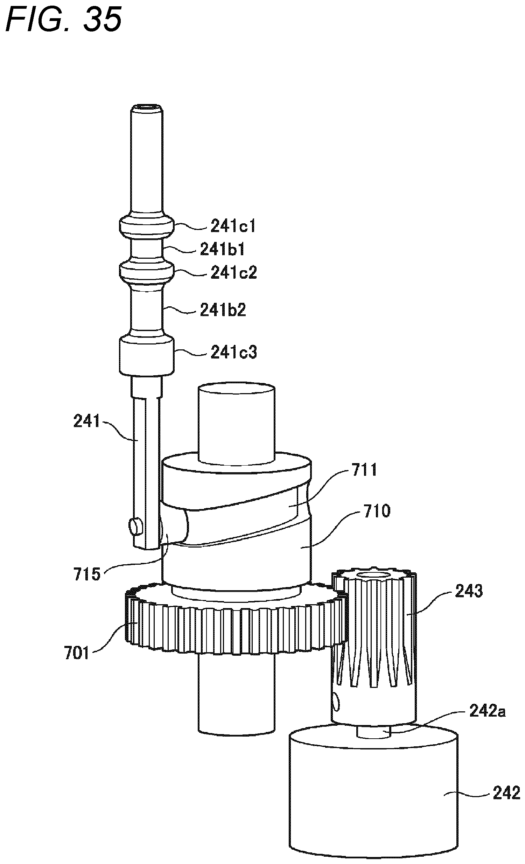

FIG. 35 is a perspective view of the drive unit of the electric prosthetic leg 1 of the fifth modification.

The drive unit of the electric prosthetic leg 1 of the fifth modification includes the servo motor 242, the pinion 243 provided on the output shaft 242a of the servo motor 242, a spur gear 701 meshing with the pinion 243, a cam drum 710 integrally rotating with the spur gear 701, and a pin member 715 provided on the lower end of the operation rod 241 and engaged with a cam groove 711 formed in the cam drum 710 and sliding along the cam groove 711. When the pinion 243 provided on the output shaft 242a of the servo motor 242 rotates and the spur gear 701 rotates, the cam drum 710 rotates accordingly, and the pin member 715 slides in the cam groove 711, and thus the operation rod 241 moves in the upper-lower direction.

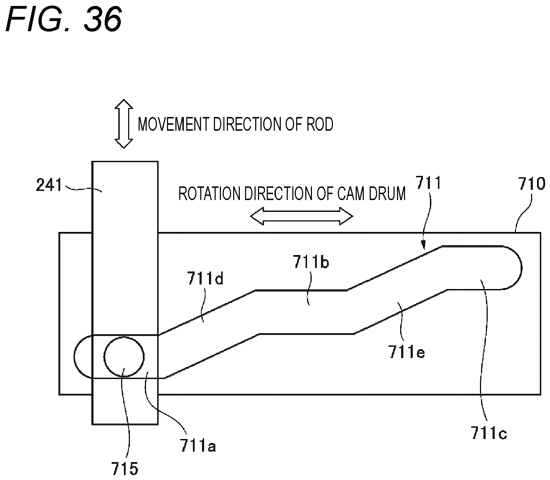

FIG. 36 is a diagram in which the cam groove 711 formed in the cam drum 710 is developed in a rotation direction.

The cam groove 711 includes a first horizontal portion 711a provided on an outer peripheral surface of the cam drum 710, a second horizontal portion 711b above the first horizontal portion 711a, a third horizontal portion 711c above the second horizontal portion 711b, a first inclined portion 711d connecting the first horizontal portion 711a and the second horizontal portion 711b, and a second inclined portion 711e connecting the second horizontal portion 711b and the third horizontal portion 711c. When the pin member 715 of the operation rod 241 is positioned at the first horizontal portion 711a, the operation rod 241 is at the position shown in (C) of FIG. 11, and when the pin member 715 is positioned at the second horizontal portion 711b, the operation rod 241 is positioned at the position shown in (B) of FIG. 11, and when the pin member 715 is positioned at the third horizontal portion 711c, the operation rod 241 is positioned at the position shown in (A) of FIG. 11. The length of each of the first horizontal portion 711a to the third horizontal portion 711c is configured to be longer than the diameter of the pin member 715.

According to the fifth modification, the position of the operation rod 241 does not change as long as the pin member 715 is positioned at the horizontal portions 711a to 711c, and therefore, the position of the operation rod 241 is maintained even when an external force is applied to the operation rod 241. Since the position of the operation rod 241 is determined by the position of the pin member 715 sliding in the cam groove 711 regardless of the rotation angle of the servo motor 242, a required accuracy of the rotation angle of the servo motor 242 is low, and a positional accuracy of the operation rod 241 is good. In addition to the relatively simple structure, a degree of freedom of an inclination angle of the cam groove 711 is large. That is, the inclination of the inclined portions 711d and 711e can be adjusted in accordance with the torque of the servo motor 242 by adjusting a diameter of the cam drum 710. Note that the spur gear 701 may be configured to transmit power to the pinion 243 provided on the output shaft 242a of the servo motor 242 through a bevel gear mechanism.

Although various embodiments have been described above with reference to the drawings, the present invention is not limited to these examples. It is apparent to those skilled in the art that various changes or modifications can be conceived within the scope described in the claims, and it is understood that the changes or modifications naturally fall within the technical scope of the present invention. In addition, respective constituent elements in the above-described embodiments may be freely combined without departing from the gist of the invention.

For example, in the above embodiment, although a prosthetic leg device (electric prosthetic leg) applied to a knee joint as an embodiment of the joint device using the connection and disconnection device of the present invention is described, the present invention is not limited thereto, and may be a prosthetic limb device (electric prosthetic limb) applied to an elbow joint, and the wearer may be an animal other than a human, or a robot. When applied to the elbow joint, the below-knee member 110 in the above embodiments becomes a distal end side of the wearer with respect to the above-knee member 120, that is, a forearm.

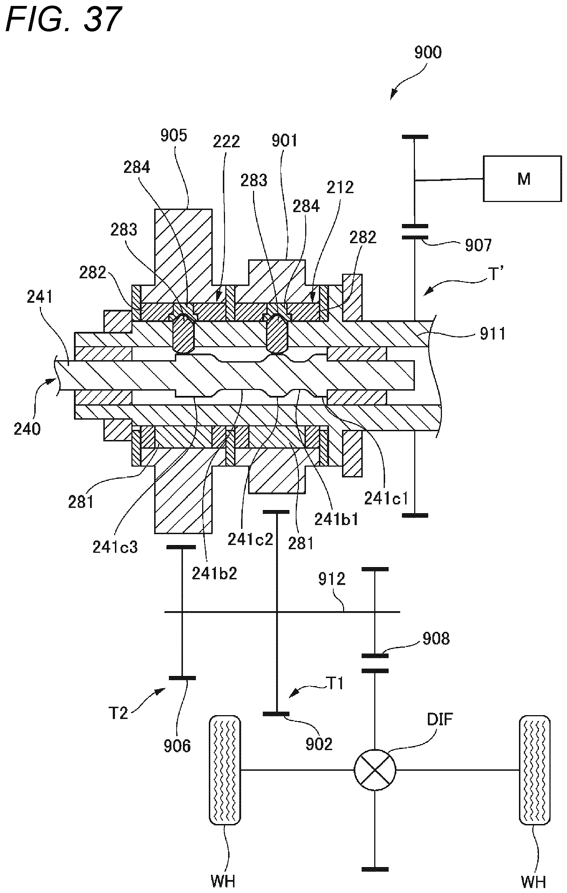

The connection and disconnection device of the present disclosure may be used not only in a joint device but also in a drive device of a vehicle. FIG. 37 is a schematic diagram of a vehicle drive device equipped with the connection and disconnection device of the above-described embodiment.

A vehicle drive device 900 in FIG. 37 includes the motor M as a drive source, a transmission T' that transmits power of the motor M, the first interrupting mechanism 210 and the second interrupting mechanism 220 provided in the transmission T', and a differential device DIF that distributes an output from the transmission T' to left and right drive wheels WH.

The transmission T' includes the first transmission mechanism T1 that transmits the power of the motor M to the left and right drive wheels WH at a first transmission ratio, and a second transmission mechanism T2 that transmits the power of the motor M to the left and right drive wheels WH at a second transmission ratio, which is different from the first transmission ratio. A relation between the first transmission ratio and the second transmission ratio is the same as that in the above-described embodiment.

The first transmission mechanism T1 includes a first drive gear 901 and a first driven gear 902 that mesh with each other. The first drive gear 901 is relatively rotatably supported by a first shaft 911, and the first driven gear 902 is integrally rotatably supported by a second shaft 912. The second transmission mechanism T2 includes a second drive gear 905 and a second driven gear 906 that mesh with each other. The second drive gear 905 is relatively rotatably supported by the first shaft 911, and the second driven gear 906 is integrally rotatably supported by the second shaft 912. In addition to the first drive gear 901 and the second drive gear 905, an input gear 907 to which the power of the motor M is input is attached to the first shaft 911 in an integrally rotatable manner. In addition to the first driven gear 902 and the second driven gear 906, an output gear 908 that can output the power of the motor M to the differential device DIF is attached to the second shaft 912 in an integrally rotatable manner.

The first interrupting mechanism 210 includes the first interrupting unit 212 provided between the first drive gear 901 and the first shaft 911, and the operation mechanism 240 that switches the first interrupting unit 212. The second interrupting mechanism 220 includes the second interrupting unit 222 provided between the second drive gear 905 and the first shaft 911, and the operation mechanism 240 that switches the second interrupting unit 222. These interrupting units 212 and 222 have a common configuration, and are configured to be switchable between the cutoff state in which power transmission is cut off, and the power transmittable state in which rotation power in both one direction and the other direction can be transmitted. Note that since the rollers 281, the operation rod 241, the pins 283, the retainer 282, and the guides 284 constituting the interrupting units 212 and 222 are the same as those in the above-described embodiment, the same reference numerals are given and the description thereof will be omitted.

In the vehicle drive device 900 configured as described above, the power of the motor M is transmitted to the left and right drive wheels WH through the second transmission mechanism T2 when the first interrupting unit 212 is in an off state and the second interrupting unit 222 is in an on state. When the first interrupting unit 212 is in the on state and the second interrupting unit 222 is in the off state, the power of the motor M is transmitted to the left and right drive wheels WH through the first transmission mechanism T1. When the first interrupting unit 212 is in the off state and the second interrupting unit 222 is in the off state, a so-called neutral state is established in which the power of the motor M is not transmitted to the left and right drive wheels WH.

By applying the connection and disconnection device of the present invention to the vehicle drive device 900, rotation adjustment during shifting is not necessary, and responsiveness during shifting is improved. In addition, the number of parts constituting the connection and disconnection device can be reduced as compared with a general dog clutch or the like. Note that the first interrupting mechanism 210 and/or the second interrupting mechanism 220 may be provided on the second shaft 912 instead of the first shaft 911. The drive wheels WH may be circular wheels as in the present embodiment, or may be starting wheels that moves a caterpillar. In the present embodiment, the connection and disconnection device is applied to the drive device that drives the drive wheels WH as an impeller that impels the vehicle, but the connection and disconnection device may be applied to a drive device that drives an impeller such as a propeller that propels other moving objects such as a ship or an aircraft. Further, in addition to the impeller of a moving object, the connection and disconnection device may be applied to a drive device that drives a working unit such as a snow removal unit or a mower unit of a working machine such as a snow removal machine or a mower.

In the present description, at least the following matters are described. Although corresponding constituent elements or the like in the embodiment described above are shown in parentheses, the present invention is not limited thereto.
(1) A connection and disconnection device (first interrupting mechanism 210, second interrupting mechanism 220), including:
   an engagement element (roller 281) disposed between a first rotation member (first driven gear 184, first driven gear 901) and a second rotation member (second shaft 182, first shaft 911); and
   an operation unit (operation rod 241, 241A, pin 283, retainer 282, guide 284) configured to operate the engagement element to be in an engagement state in which the first rotation member and the second rotation member are integrally rotatable or a non-engagement state in which the first rotation member and the second rotation member are relatively rotatable, in which
   the operation unit includes
      an actuator (retainer 282, guide 284) configured to move the engagement element, and
      an operator (operation rod 241, 241A, pin 283) configured to be able to operate the engagement element via the actuator or to operate the engagement element not via the actuator,
   the first rotation member and the second rotation member are arranged such that
      rotation axes of the first rotation member and the second rotation member coincide with each other, and
      the first rotation member and the second rotation member at least partially overlap each other when viewed in an orthogonal direction orthogonal to the rotation axes,
   the operator includes
      an advancing and retracting element (pin 283) that is provided to be capable of advancing and retracting along the orthogonal direction orthogonal to the rotation axes, and
      an extension portion (operation rod 241, 241A, rack 241a) that extends along the rotation axes and is provided to be capable of advancing and retracting along the rotation axes, and
   the advancing and retracting element is provided such that an inner end thereof, which is an end on a side closer to the rotation axes in the orthogonal direction, is in contact with the extension portion.

According to (1), it is possible to appropriately switch the engagement element between the engagement state and the non-engagement state by the operation unit.

(2) The connection and disconnection device according to (1), in which
the extension portion is mechanically connected to a drive unit (servo motor 242) that drives the extension portion, and
the extension portion includes a first extension portion (operation rod 241, 241A) and a second extension portion (rack 241a) that are provided in a manner of being rotatable relative to each other about the rotation axes.

According to (2), since the extension portion includes the first extension portion and the second extension portion which are provided in a manner of being rotatable relative to each other, a force acting on the extension portion during the retracting movement of the advancing and retracting element becomes the rotation force of the extension portion, and the operation load of the extension portion can be reduced.

(3) The connection and disconnection device according to (2), in which
the drive unit is provided to make the extension portion advance and retract in a direction along the rotation axis.

According to (3), the extension portion can advance and retract in the rotation axis direction by the drive unit.

(4) The connection and disconnection device according to (3), in which
when one of the first extension portion and the second extension portion that is disposed closer to the drive unit is defined as the second extension portion,
the first extension portion is provided in a manner of being rotatable with respect to the rotation axis and being capable of advancing and retracting in the direction along the rotation axis, and
the second extension portion is provided in a manner of being non-rotatable with respect to the rotation axis and being capable of advancing and retracting in the direction along the rotation axis.

According to (4), by making the first extension portion rotatable and making the second extension portion disposed closer to the drive unit non-rotatable, power is appropriately input from the drive unit to the second extension portion.

(5) The connection and disconnection device according to any one of (1) to (4), in which
the first rotation member has a larger diameter than the second rotation member and is formed in a hollow shape so as to have a first internal space therein,
the second rotation member is at least partially positioned in the first internal space and is formed in a hollow shape so as to have a second internal space (internal space S2) therein, and
the extension portion is disposed so as to be partially positioned in the second internal space.

According to (5), it is possible to prevent foreign matters from entering the second internal space.

(6) The connection and disconnection device according to (5), further including:
a closing portion (lid member 188) having an insertion hole through which the extension portion is inserted, the closing portion being provided so as to close one end of the second internal space in a direction along the rotation axis.

According to (6), it is possible to prevent foreign matters from entering the second internal space.

(7) The connection and disconnection device according to (6), in which
the closing portion is provided to be attachable to and detachable from the second rotation member.

According to (7), the extension portion can be easily replaced, and the optimal extension portion can be used in accordance with the timing of the on and off state of the connection and disconnection device.

(8) The connection and disconnection device according to (7), further including:
a bearing portion (bearing B5) disposed in a vicinity of the closing portion of the second internal space and pivotally supporting the extension portion.

According to (8), by disposing the bearing portion in the vicinity of the closing portion, the bearing is also easily attached and detached when replacing the extension portion.

(9) The connection and disconnection device according to any one of (5) to (8), in which
the extension portion is provided to be rotatable about the rotation axis.

According to (9), since the extension portion is rotatable around the rotation axis, a force acting on the extension portion during the retracting movement of the advancing and retracting element becomes the rotation force of the extension portion, and the operation load of the extension portion can be reduced.

(10) The connection and disconnection device according to any one of (1) to (9), in which
the extension portion is mechanically connected to a drive unit that drives the extension portion,
the drive unit includes a drive source (servo motor 242) and an elastic energy accumulating mechanism (elastic energy accumulating mechanism 300, 300A), and
the elastic energy accumulating mechanism includes a first drive member (first rotation bracket 310, second operation rod 342), a second drive member (second rotation bracket 320, third operation rod 343), a first elastic member (first elastic member 331, fourth elastic member 334) interposed between the first drive member and the second drive member and configured to be able to transmit power, and a second elastic member (second elastic member 332, third elastic member 333) interposed between the second drive member and a third drive member (pinion 243, first operation rod 341) or the extension portion and configured to be able to transmit power.

According to (10), the power consumption of the drive source can be reduced.

(11) The connection and disconnection device according to (10), in which
the power of the drive source in one direction is set to be transmitted from the first drive member to the second drive member via the first elastic member, and transmitted from the second drive member to the third drive member or the extension portion, and
the power of the drive source in another direction is set to be transmitted from the first drive member to the second drive member, and transmitted from the second drive member to the third drive member or the extension portion via the second elastic member.

According to (11), the power consumption of the drive source can be reduced.

(12) The connection and disconnection device according to (11), in which
the drive source is configured to be able to generate rotation power,
the first drive member (first rotation bracket 310) and the second drive member (second rotation bracket 320) are provided as rotors having the same rotation axis,
the first elastic member (first elastic member 331) is configured to transmit rotation power input to the first drive member to the second drive member, and
the second elastic member (second elastic member 332) is configured to transmit rotation power transmitted to the second drive member to the third drive member (pinion 243) or the extension portion.

According to (12), energy can be accumulated using the rotation power.

(13) The connection and disconnection device according to (11), in which
the drive source is configured to generate translation power,
the first drive member (second operation rod 342) and the second drive member (third operation rod 343) are provided as rotors having the same translation axis,
the first elastic member (forth elastic member 334) is configured to transmit translation power input to the first drive member to the second drive member, and
the second elastic member (third elastic member 333) is configured to transmit translation power transmitted to the second drive member to the third drive member (first operation rod 341) or the extension portion.

According to (13), energy can be accumulated using the translation power.

(14) The connection and disconnection device according to any one of (1) to (13), in which
the extension portion is mechanically connected to a drive unit that drives the extension portion,
the drive unit includes a drive source (servo motor 242) and a power conversion mechanism,
the power conversion mechanism includes a fourth drive member (arm portion 400, Geneva driven gear 610, cam drum 710), and
the fourth drive member is arranged to engage with a fifth drive member (coupling portion 500, Geneva driven gear 620) or the extension portion in a power transmittable manner.

According to (14), the power of the drive source can be appropriately transmitted.

(15) The connection and disconnection device according to (14), in which
the drive source is configured to be able to generate rotation power, and
the power conversion mechanism is configured to convert rotation power input to the fourth drive member (arm portion 400, cam drum 710) into translation power and transmit the translation power to the fifth drive member (coupling portion 500) or the extension portion.

According to (15), the rotation power of the drive source can be converted into translation power and then transmitted.

(16) The connection and disconnection device according to (15), in which
one of the fourth drive member and the fifth drive member or one of the fourth drive member and the extension portion is provided with a guide groove (long hole 505, cam groove 711), and
another of the fourth drive member and the fifth drive member or another of the fourth drive member and the extension portion is provided with an engagement projection (engagement pin 421, pin member 715) guided by the guide groove.

According to (16), the rotation power can be converted into the translation power by the engagement projection engaged with the guide groove.

(17) The connection and disconnection device according to (14), in which
the drive source is configured to be able to generate rotation power, and
the power conversion mechanism is configured to convert rotation power (continuous rotation) input to the fourth drive member (Geneva driven gear 610) into another rotation power (intermittent rotation) and transmit the another rotation power to the fifth drive member or the extension portion.

According to (17), the input rotation power can be converted into another kind of rotation power and then transmitted.

(18) The connection and disconnection device according to any one of (14) to (17), in which
the drive unit includes a third elastic member (torsion spring 402) interposed between the drive source and the fourth drive member and configured to be able to transmit power.

According to (18), the load of the drive source can be reduced.

(19) The connection and disconnection device according to any one of (1) to (18), in which
the first rotation member has a larger diameter than the second rotation member and is formed in a hollow shape so as to have a first internal space therein,
the second rotation member is at least partially positioned in the first internal space and is formed in a hollow shape so as to have a second internal space therein, and
the extension portion is disposed so as to be at least partially positioned in the second internal space.

According to (19), it is possible to reduce the size.

(20) The connection and disconnection device according to any one of (1) to (19), in which
when the advancing and retracting element is positioned on an outer side in the orthogonal direction, the engagement element is in one of the engagement state and the non-engagement state, and
when the advancing and retracting element is positioned on an inner side in the orthogonal direction, the engagement element is in another of the engagement state and the non-engagement state.

According to (20), the engagement state and the non-engagement state can be controlled according to the position of the advancing and retracting element in the radial direction.

(21) The connection and disconnection device according to (20), in which
when the operator is at a first position in a direction along the rotation axis, the advancing and retracting element is positioned on the outer side in the orthogonal direction, and
when the operator is at a second position in the direction along the rotation axis, the advancing and retracting element is positioned on the inner side in the orthogonal direction.

According to (21), by making the operator advance and retract to the first position or the second position along the rotation axis, it is possible to control the position of the advancing and retracting element in the radial direction.

(22) The connection and disconnection device according to (21), in which
the engagement element is in the non-engagement state when the advancing and retracting element is positioned on the outer side in the orthogonal direction, and
when the extension portion is positioned at a third position different from the first position and the second position in the direction along the rotation axis, the advancing and retracting element is positioned on the outer side in the orthogonal direction.

According to (22), by moving the operator to the third position along the rotation axis, it is possible to control the position of the advancing and retracting element in the radial direction.

(23) The connection and disconnection device according to any one of (1) to (22), in which
the engagement element includes a plurality of engagement bodies (roller 281) arranged to be spaced apart from each other in a circumferential direction around the rotation axis, and
the actuator includes
   a plurality of the advancing and retracting elements arranged to be spaced apart from each other in the circumferential direction around the rotation axis and configured to move the plurality of engagement bodies, and
   a retainer (retainer 282, guide 284) retaining the plurality of engagement bodies and the plurality of advancing and retracting elements.

According to (23), the plurality of advancing and retracting elements and the retainer constitute the actuator.

(24) The connection and disconnection device according to any one of (1) to (22), in which
the engagement element includes
   a plurality of engagement bodies (roller 281) arranged to be spaced apart from each other in a circumferential direction around the rotation axis, and
   a retainer (retainer 282, guide 284) retaining the plurality of engagement bodies, and
the advancing and retracting element is configured to move the plurality of engagement elements via the retainer.

According to (24), the plurality of engagement bodies and the retainer constitute the engagement element.

(25) The connection and disconnection device according to any one of (1) to (24), further including:
a third rotation member (second driven gear 186) and a fourth rotation member (second shaft 182) arranged such that rotation axes thereof coincide with the rotation axis;
another engagement element (roller 281) disposed between the third rotation member and the fourth rotation member; and
another operation unit (operation rod 241, 241A, pin 283, retainer 282, guide 284) configured to operate the another engagement element to be in an engagement state in which the third rotation member and the fourth rotation member are integrally rotatable or a non-engagement state in which the third rotation member and the fourth rotation member are relatively rotatable.

According to (25), the engagement state of the third rotation member and the fourth rotation member and the non-engagement state of the third rotation member and the fourth rotation member can be appropriately switched by the other operation unit.

(26) The connection and disconnection device according to (25), in which
the another operation unit includes
another actuator (retainer 282, guide 284) configured to move the another engagement element, and
another operator (operation rod 241, 241A, pin 283) configured to be able to operate the another engagement element via the another actuator or configured to be able to operate the another engagement element not via the another actuator.

According to (26), the engagement state of the third rotation member and the fourth rotation member and the non-engagement state of the third rotation member and the fourth rotation member can be appropriately switched by the other operator.

(27) The connection and disconnection device according to (26), in which
the another operator includes
   another advancing and retracting element (pin 283) configured to be able to advance and retract along the orthogonal direction orthogonal to the rotation axis, and
   another extension portion (operation rod 241, 241A, rack 241a) extending along the rotation axis and configured to be able to advance and retract along the rotation axis, and
the another advancing and retracting element is provided such that an inner end thereof, which is an end on a side closer to the rotation axis in the orthogonal direction, comes into contact with the another extension portion.

According to (27), in the other operator, the inner end of the other advancing and retracting element is in contact with the extension portion, and thus the other advancing and retracting element can advance and retract along the orthogonal direction orthogonal to the rotation axes of the third rotation member and the fourth rotation member.

(28) The connection and disconnection device according to (27), in which
the second rotation member and the fourth rotation member are configured to be integrally rotatable, and
the extension portion and the another extension portion are integrally provided, and are arranged so as to be at different positions in a direction along the rotation axis.

According to (28), the drive source can be used in common, and the control can be further simplified.

(29) A joint device with the connection and disconnection device according to any one of (1) to (28), the joint device including:
a first member (below-knee member 110);
a second member (above-knee member 120);
a connecting unit (knee joint mechanism 130) that connects the first member and the second member such that an angle formed by the first member and the second member is changeable; and
an enlarging and reducing device (enlarging and reducing device 200) configured to be able to enlarge and reduce the angle formed by the first member and the second member, in which
the enlarging and reducing device includes a power source (motor M) and a power transmission unit (transmission T) configured to transmit power of the power source, and
the connection and disconnection device is applied to the power transmission unit.

According to (29), the engagement state and the non-engagement state of the engagement element can be appropriately switched in the joint device.

(30) The joint device according to (29), in which
the joint device is a prosthetic limb device (electric prosthetic leg 1),
the first member is attached to an wearer so as to be closer to a distal end side of the wearer than the second member, and
the connecting unit is configured to function as a joint of the wearer.

According to (30), it is possible to appropriately switch the engagement state and the non-engagement state of the engagement element in the prosthetic limb device.

(31) The joint device according to (30), in which
the prosthetic limb device is a prosthetic leg device (electric prosthetic leg 1) configured to be attached to a leg of the wearer.

According to (31), it is possible to appropriately switch the engagement state and the non-engagement state of the engagement element in the prosthetic leg device.

(32) The joint device according to (31), in which
the second member of the prosthetic leg device is configured to be attached to a thigh of the leg, and
the connecting unit is configured to function as a knee joint between the thigh and a lower leg.

According to (32), it is possible to appropriately switch the engagement state and the non-engagement state of the engagement elements in the knee joint of the prosthetic leg device.

Although various embodiments have been described above, the present invention is not limited to these examples. It is apparent that those skilled in the art may conceive of various modifications and changes within the scope described in the claims, and it is understood that such modifications and changes naturally fall within the technical scope of the present invention. In addition, constituent elements in the embodiment described above may be freely combined without departing from the gist of the present invention.

Note that the present application is based on a Japanese patent application (Japanese Patent Application No. 2021-203497) filed on December 15, 2021, and the contents thereof are incorporated herein as a reference.

### REFERENCE SIGNS LIST

1 electric prosthetic leg (joint device, prosthetic limb device, prosthetic leg device)
110 below-knee member (first member)
120 above-knee member (second member)
130 knee joint mechanism (connecting unit)
182 second shaft (second rotation member, fourth rotation member)
184 first driven gear (first rotation member)
186 second driven gear (third rotation member)
188 lid member (closing portion)
200 enlarging and reducing device
210 first interrupting mechanism (connection and disconnection device)
220 second interrupting mechanism (connection and disconnection device)
241, 241A operation rod (operation unit, operator, extension portion, first extension portion)
241a rack (extension portion, second extension portion, rack portion)
242 servo motor (drive unit, drive source)
243 pinion (third drive member)
281 roller (engagement element, engagement body)
282 retainer (operation unit, actuator, retainer)
283 pin (operation unit, operator, advancing and retracting element)
284 guide (operation unit, actuator, retainer)
300, 300A elastic energy accumulating mechanism
310 first rotation bracket (first drive member)
320 second rotation bracket (second drive member)
331 first elastic member
332 second elastic member
333 third elastic member (second elastic member)
334 fourth elastic member (first elastic member)
341 first operation rod (third drive member)
342 second operation rod (first drive member)
343 third operation rod (second drive member)
400 arm portion (fourth drive member)
402 torsion spring (third elastic member)
421 engagement pin (engagement projection)
500 coupling portion (fifth drive member)
505 long hole (guide groove)
610 Geneva drive gear
612 drive pin
620 Geneva driven gear (fifth drive member)
710 cam drum (fourth drive member)
711 cam groove
715 pin member (engagement projection)
901 first drive gear (first rotation member)
911 first shaft (second rotation member)
B5 bearing (bearing portion)
S2 internal space (second internal space)
T transmission (power transmission unit)
M motor (power source)

## Claims

1. A connection and disconnection device, comprising:
an engagement element disposed between a first rotation member and a second rotation member; and
an operation unit configured to operate the engagement element to be in an engagement state in which the first rotation member and the second rotation member are integrally rotatable or a non-engagement state in which the first rotation member and the second rotation member are relatively rotatable, wherein
the operation unit includes
an actuator configured to move the engagement element, and
an operator configured to be able to operate the engagement element via the actuator or to be able to operate the engagement element not via the actuator,
the first rotation member and the second rotation member are arranged such that
rotation axes of the first rotation member and the second rotation member coincide with each other, and
the first rotation member and the second rotation member at least partially overlap each other when viewed in an orthogonal direction orthogonal to the rotation axes,
the operator includes
an advancing and retracting element that is provided to be capable of advancing and retracting along the orthogonal direction orthogonal to the rotation axes, and
an extension portion that extends along the rotation axes and is provided to be capable of advancing and retracting along the rotation axes, and
the advancing and retracting element is provided such that an inner end thereof, which is an end on a side closer to the rotation axes in the orthogonal direction, is in contact with the extension portion.

2. The connection and disconnection device according to claim 1, wherein
the extension portion is mechanically connected to a drive unit that drives the extension portion, and
the extension portion includes a first extension portion and a second extension portion that are provided in a manner of being rotatable relative to each other about the rotation axes.

3. The connection and disconnection device according to claim 2, wherein
the drive unit is provided to make the extension portion advance and retract in a direction along the rotation axis.

4. The connection and disconnection device according to claim 3, wherein
when one of the first extension portion and the second extension portion that is disposed closer to the drive unit is defined as the second extension portion,
the first extension portion is provided in a manner of being rotatable with respect to the rotation axis and being capable of advancing and retracting in the direction along the rotation axis, and
the second extension portion is provided in a manner of being non-rotatable with respect to the rotation axis and being capable of advancing and retracting in the direction along the rotation axis.

5. The connection and disconnection device according to any one of claims 1 to 4, wherein
the first rotation member has a larger diameter than the second rotation member and is formed in a hollow shape so as to have a first internal space therein,
the second rotation member is at least partially positioned in the first internal space and is formed in a hollow shape so as to have a second internal space therein, and
the extension portion is disposed so as to be partially positioned in the second internal space.

6. The connection and disconnection device according to claim 5, further comprising:
a closing portion having an insertion hole through which the extension portion is inserted, the closing portion being provided so as to close one end of the second internal space in a direction along the rotation axis.

7. The connection and disconnection device according to claim 6, wherein
the closing portion is provided to be attachable to and detachable from the second rotation member.

8. The connection and disconnection device according to claim 7, further comprising:
a bearing portion disposed in a vicinity of the closing portion of the second internal space and pivotally supporting the extension portion.

9. The connection and disconnection device according to any one of claims 5 to 8, wherein
the extension portion is provided to be rotatable about the rotation axis.

10. The connection and disconnection device according to any one of claims 1 to 9, wherein
the extension portion is mechanically connected to a drive unit that drives the extension portion,
the drive unit includes a drive source and an elastic energy accumulating mechanism, and
the elastic energy accumulating mechanism includes a first drive member, a second drive member, a first elastic member interposed between the first drive member and the second drive member and configured to be able to transmit power, and a second elastic member interposed between the second drive member and a third drive member or the extension portion and configured to be able to transmit power.

11. The connection and disconnection device according to claim 10, wherein
the power of the drive source in one direction is set to be transmitted from the first drive member to the second drive member via the first elastic member, and transmitted from the second drive member to the third drive member or the extension portion, and
the power of the drive source in another direction is set to be transmitted from the first drive member to the second drive member, and transmitted from the second drive member to the third drive member or the extension portion via the second elastic member.

12. The connection and disconnection device according to claim 11, wherein
the drive source is configured to be able to generate rotation power,
the first drive member and the second drive member are provided as rotors having the same rotation axis,
the first elastic member is configured to transmit rotation power input to the first drive member to the second drive member, and
the second elastic member is configured to transmit rotation power transmitted to the second drive member to the third drive member or the extension portion.

13. The connection and disconnection device according to claim 11, wherein
the drive source is configured to generate translation power,
the first drive member and the second drive member are provided as rotors having the same translation axis,
the first elastic member is configured to transmit translation power input to the first drive member to the second drive member, and
the second elastic member is configured to transmit translation power transmitted to the second drive member to the third drive member or the extension portion.

14. The connection and disconnection device according to any one of claims 1 to 13, wherein
the extension portion is mechanically connected to a drive unit that drives the extension portion,
the drive unit includes a drive source and a power conversion mechanism,
the power conversion mechanism includes a fourth drive member, and
the fourth drive member is arranged to engage with a fifth drive member or the extension portion in a power transmittable manner.

15. The connection and disconnection device according to claim 14, wherein
the drive source is configured to be able to generate rotation power, and
the power conversion mechanism is configured to convert rotation power input to the fourth drive member into translation power and transmit the translation power to the fifth drive member or the extension portion.

16. The connection and disconnection device according to claim 15, wherein
one of the fourth drive member and the fifth drive member or one of the fourth drive member and the extension portion is provided with a guide groove, and
another of the fourth drive member and the fifth drive member or another of the fourth drive member and the extension portion is provided with an engagement projection guided by the guide groove.

17. The connection and disconnection device according to claim 14, wherein
the drive source is configured to be able to generate rotation power, and
the power conversion mechanism is configured to convert rotation power input to the fourth drive member into another rotation power and transmit the another rotation power to the fifth drive member or the extension portion.

18. The connection and disconnection device according to any one of claims 14 to 17, wherein
the drive unit includes a third elastic member interposed between the drive source and the fourth drive member and configured to be able to transmit power.

19. The connection and disconnection device according to any one of claims 1 to 18, wherein
the first rotation member has a larger diameter than the second rotation member and is formed in a hollow shape so as to have a first internal space therein,
the second rotation member is at least partially positioned in the first internal space and is formed in a hollow shape so as to have a second internal space therein, and
the extension portion is disposed so as to be at least partially positioned in the second internal space.

20. The connection and disconnection device according to any one of claims 1 to 19, wherein
when the advancing and retracting element is positioned on an outer side in the orthogonal direction, the engagement element is in one of the engagement state and the non-engagement state, and
when the advancing and retracting element is positioned on an inner side in the orthogonal direction, the engagement element is in another of the engagement state and the non-engagement state.

21. The connection and disconnection device according to claim 20, wherein
when the operator is at a first position in a direction along the rotation axis, the advancing and retracting element is positioned on the outer side in the orthogonal direction, and
when the operator is at a second position in the direction along the rotation axis, the advancing and retracting element is positioned on the inner side in the orthogonal direction.

22. The connection and disconnection device according to claim 21, wherein
the engagement element is in the non-engagement state when the advancing and retracting element is positioned on the outer side in the orthogonal direction, and
when the extension portion is positioned at a third position different from the first position and the second position in the direction along the rotation axis, the advancing and retracting element is positioned on the outer side in the orthogonal direction.

23. The connection and disconnection device according to any one of claims 1 to 22, wherein
the engagement element includes a plurality of engagement bodies arranged to be spaced apart from each other in a circumferential direction around the rotation axis, and
the actuator includes
a plurality of the advancing and retracting elements arranged to be spaced apart from each other in the circumferential direction around the rotation axis and configured to move the plurality of engagement bodies, and
a retainer retaining the plurality of engagement bodies and the plurality of advancing and retracting elements.

24. The connection and disconnection device according to any one of claims 1 to 22, wherein
the engagement element includes
a plurality of engagement bodies arranged to be spaced apart from each other in a circumferential direction around the rotation axis, and
a retainer retaining the plurality of engagement bodies, and
the advancing and retracting element is configured to move the plurality of engagement elements via the retainer.

25. The connection and disconnection device according to any one of claims 1 to 24, further comprising:
a third rotation member and a fourth rotation member arranged such that rotation axes thereof coincide with the rotation axis;
another engagement element disposed between the third rotation member and the fourth rotation member; and
another operation unit configured to operate the another engagement element to be in an engagement state in which the third rotation member and the fourth rotation member are integrally rotatable or a non-engagement state in which the third rotation member and the fourth rotation member are relatively rotatable.

26. The connection and disconnection device according to claim 25, wherein
the another operation unit includes
another actuator configured to move the another engagement element, and
another operator configured to be able to operate the another engagement element via the another actuator or configured to be able to operate the another engagement element not via the another actuator.

27. The connection and disconnection device according to claim 26, wherein
the another operator includes
another advancing and retracting element configured to be able to advance and retract along the orthogonal direction orthogonal to the rotation axis, and
another extension portion extending along the rotation axis and configured to be able to advance and retract along the rotation axis, and
the another advancing and retracting element is provided such that an inner end thereof, which is an end on a side closer to the rotation axis in the orthogonal direction, comes into contact with the another extension portion.

28. The connection and disconnection device according to claim 27, wherein
the second rotation member and the fourth rotation member are configured to be integrally rotatable, and
the extension portion and the another extension portion are integrally provided, and are arranged so as to be at different positions in a direction along the rotation axis.

29. Ajoint device with the connection and disconnection device according to any one of claims 1 to 28, the joint device comprising:
a first member;
a second member;
a connecting unit that connects the first member and the second member such that an angle formed by the first member and the second member is changeable; and
an enlarging and reducing device configured to be able to enlarge and reduce the angle formed by the first member and the second member, wherein
the enlarging and reducing device includes a power source and a power transmission unit configured to transmit power of the power source, and
the connection and disconnection device is applied to the power transmission unit.

30. The joint device according to claim 29, wherein
the joint device is a prosthetic limb device,
the first member is attached to a wearer so as to be closer to a distal end side of the wearer than the second member, and
the connecting unit is configured to function as a joint of the wearer.

31. The joint device according to claim 30, wherein
the prosthetic limb device is a prosthetic leg device configured to be attached to a leg of the wearer.

32. The joint device according to claim 31, wherein
the second member of the prosthetic leg device is configured to be attached to a thigh of the leg, and
the connecting unit is configured to function as a knee joint between the thigh and a lower leg.
